# EUROPEAN PATENT APPLICATION

(11) **EP 4 424 327 A1**
(43) Date of publication of application: **04.09.2024**
(21) Application number: 22887119.0
(22) Date of filing: 27.10.2022
(51) Int. Cl.: A61K 45/00, A61K 31/165, A61K 31/167, A61K 31/18, A61K 31/343, A61K 31/40, A61K 31/506, A61P 1/00, A61P 3/10, A61P 13/12, A61P 17/00, A61P 25/00, A61P 27/02

(54) **METHOD OR AGENT WITH HDAC REGULATOR, FOR TREATMENT OF DIABETES AND COMPLICATIONS**

(30) Priority: 29.10.2021 JP 2021177845
(71) Applicant: Biozipcode, Inc., Otsu-shi, Shiga 520-2121 (JP)
(72) Inventor: KOJIMA, Hideto, Otsu-shi, Shiga 520-2192 (JP); KATAGI, Miwako, Otsu-shi, Shiga 520-2192 (JP)
(74) Representative: Engelhard, Markus
(86) International application number: PCT/JP2022/040140
(87) International publication number: WO 2023/074794

(57) **Abstract**

The present disclosure provides a novel treatment of diabetes mellitus. The present disclosure provides a treatment of diabetes mellitus by combining control of blood glucose and HDAC regulation. In one embodiment, the present disclosure provides a treatment of diabetes mellitus and/or a disease, disorder, and/or symptom associated with diabetes mellitus by combining control of blood glucose and HDAC regulation. In one embodiment, the diabetes mellitus and/or the disease, disorder, and/or symptom associated with diabetes mellitus is treated by targeting aberrant stem cells through a combination of control of blood glucose and HDAC regulation.

## Description

### Technical Field

The present disclosure relates to a treatment and prevention of diabetes mellitus and/or an associated disease thereof by combining histone deacetylase (HDAC) regulation and blood glucose control. More particularly, the present disclosure relates to a treatment and prevention of diabetes mellitus and/or an associated disease thereof by regulating HDACs in a subject with blood glucose control and/or regulating HDACs in combination with a blood glucose control treatment.

### Background Art

Diabetes mellitus is generally classified into type 1 and type 2 (Non Patent Literature 1). However, since details of the onset of both types are unknown, classification into type 1 and type 2 itself should be considered as lacking validity as classification based on the cause of the diseases. In addition, currently, while various drugs have been developed for a treatment of diabetes mellitus, many of those drugs are intended for blood glucose control, which can be effective for prevention of progression of diabetes mellitus, but can be insufficient for curing diabetes mellitus. Moreover, diabetic complications such as a neurological disorder, nephropathy, a hepatic disorder, retinopathy, fatty liver, a gastrointestinal disorder, delayed fracture healing, an eating disorder, and a skin disorder also develop as common complications of diabetes mellitus classified into type 1 and type 2. These complications can be difficult to cure once they develop.

### Citation List

### Non Patent Literature

Non Patent Literature 1: Japanese Clinical Practice Guideline for Diabetes 2016, the Japan Diabetes Society, Nankodo Co., Ltd.

### Summary of Invention

### Solution to Problem

The present inventors have found treatment strategies for diabetes mellitus and/or an associated disease thereof based on HDAC regulation in combination with blood glucose control are significantly effective. Based on the novel finding, the present disclosure provides means for treating diabetes mellitus and/or an associated disease thereof.

Accordingly, the present disclosure provides the following.

### (Item 1)

A composition containing a histone deacetylase (HDAC) regulator for a treatment and/or prevention of diabetes mellitus and/or a disease, disorder, and/or symptom associated with diabetes mellitus in a subject, in which the composition is administered in a controlled blood glucose state of the subject.

### (Item 2)

A composition containing a histone deacetylase (HDAC) regulator for a treatment and/or prevention of diabetes mellitus and/or a disease, disorder, and/or symptom associated with diabetes mellitus in a subject, in which the composition is administered in combination with a blood glucose control treatment.

### (Item 3)

The composition according to any one of the items, in which the HDAC regulator inhibits at least one of class I, class II, and class IV HDACs.

### (Item 4)

The composition according to any one of the items, in which the HDAC regulator includes at least one selected from the group consisting of fingolimod (FTY720), givinostat, vorinostat (suberoylanilide hydroxamic acid, SAHA), belinostat, panobinostat, resminostat, abexinostat, mocetinostat, trichostatin A (TSA), romidepsin, apicidin, chidamide, entinostat, tacedinaline, tucidinostat, pracinostat (SB939), quisinostat, tefinostat, rocilinostat, fimepinostat, nanatinostat, domatinostat, trapoxin (TPX), cyclic hydroxamic acid-containing peptide 1 (CHAP1), valproate (VPA), sodium butyrate (NaBu), 4-phenylbutyric acid, OBP-801, ME-344, CG200745, MPT0E014, RGFP966 (MCE, HY-13909), SK-7041, scriptaid, MC1568, MHY2256, ricolinostat (ACY-1215), Tubacin (S2239), Tubathian A, Tubastatin A, Pojamide, AN7, CUDC-101, KD5170, R36465, SOU-HDAC42, LBH589, SFN, phenyl-hexyl isothiocyanate, dacinostat (LAQ824), m-carboxycinnamic acid bis-hydroxamide (CBHA), azelaoyl-bis-hydroxamic acid (ABHA) (N,N'-dihydroxynonanediamide), AR-42, suberoyl bis-hydroxamic acid (SBHA), (pivaloyloxy)methyl butyrate (pivanex, AN-9), diallyl trisulfide (DATS), PCI-24781, nicotinamide, tenovin-1, tenovin-6, sirtinol, EX-527, HBI-8000, kevetrin, CHR-3996, 4SC202, WW437, α-ketoamides, heterocyclic ketones, cambinol, I-7ab, C149, depudecin, SEN 196, COMPOUND6J, JGB 1741, JQ1, I-BET 151, BY27, CUDC907, DWP0016, MC1568, W2, RGFP109, tinostamustine (EDO-S101), J22352, M344, ITF-3056, TMP195 (TFMO 2), MI192, Santacruzamate A(CAY10683), ACY-738, BML-281 (CAY10603), LTB2, CKD-506, WK2-16, PCI-34051, ESM-HDAC528, Merck60, (N-hydroxy-7-2-naphthylthio)heptanomide (HNHA), KBH-A42, AGK2, sulforaphane, MS-275, resveratrol, APHA, curcumin, remetinostat, citarinostat, dacinostat, droxinostat, BRD3308, nimbolide, betaine, and chrysophanol.

### (Item 5)

The composition according to any one of the items, in which the HDAC regulator includes at least one selected from the group consisting of fingolimod (FTY720), givinostat, trichostatin A, vorinostat, belinostat, panobinostat, resminostat, abexinostat, quisinostat, pracinostat, rocilinostat, nanatinostat, dacinostat, (N-hydroxy-7-2-naphthylthio)heptanomide, valproate, CG200745, scriptaid, KBH-A42, romidepsin, sodium butyrate, 4-phenylbutyric acid, SK-7041, WW437, entinostat, tacedinaline, mocetinostat, (pivaloyloxy)methyl butyrate, trapoxin, α-ketoamides, heterocyclic ketones, diallyl trisulfide, ricolinostat, Tubacin, azelaoyl-bis-hydroxamic acid, and m-carboxycinnamic acid bis-hydroxamide.

### (Item 6)

The composition according to any one of the items, in which the control of blood glucose is performed by at least one selected from the group consisting of administration of a hypoglycemic agent, dietary restriction, and nutrient infusion control.

### (Item 7)

The composition according to any one of the items, in which the hypoglycemic agent is selected from the group consisting of insulin, an insulin analog, a biguanide, an insulin secretagogue (a sulfonylurea drug (SU drug) or the like), a fast-acting insulin secretagogue (a glinide drug or the like), an incretin-related drug (a DPP-4 inhibitor, a GLP-1 receptor agonist, or the like), a sugar absorption regulator (α-glucosidase inhibitor (α-GI)), an insulin resistance-ameliorating agent (a thiazolidine drug), a urine glucose evacuant (a SGLT2 inhibitor), and a combination of two or more thereof.

### (Item 8)

The composition according to any one of the items, in which the control of blood glucose includes controlling fasting blood glucose to less than 126 mg/dL, controlling a blood glucose level at 2 hours of an oral glucose tolerance test to less than 200 mg/dL, or controlling a casual blood glucose level to less than 200 mg/dL.

### (Item 9)

The composition according to any one of the items, in which the composition is used for treating diabetes mellitus and/or a disease, disorder, and/or symptom associated with diabetes mellitus.

### (Item 10)

The composition according to any one of the items, in which the disease, disorder, and/or symptom includes diabetic complications.

### (Item 11)

The composition according to any one of the items, in which the disease, disorder, and/or symptom is selected from the group consisting of a neurological disorder, nephropathy, a hepatic disorder, retinopathy, fatty liver, a gastrointestinal disorder, delayed fracture healing, an eating disorder, and a skin disorder.

### (Item 12)

The composition according to any one of the items, in which the subject is diagnosed as having abnormal hematopoietic stem cells (HSCs) or cells derived therefrom.

### (Item 13)

The composition according to any one of the items, in which the abnormal hematopoietic stem cells have at least one of variable levels of HDAC and increased levels of CD 106.

### (Item 14)

The composition according to any one of the items, in which the composition is a pharmaceutical composition.

### (Item 15)

The composition according to any one of the items, further containing a pharmaceutically acceptable excipient.

### (Item 16)

A method for treating and/or preventing diabetes mellitus and/or a disease, disorder, and/or symptom associated with diabetes mellitus in a subject, the method including administering an effective amount of a histone deacetylase (HDAC) regulator to the subject in a controlled blood glucose state.

### (Item 17)

A method for treating and/or preventing diabetes mellitus and/or a disease, disorder, and/or symptom associated with diabetes mellitus in a subject, the method including: administering an effective amount of a histone deacetylase (HDAC) regulator; and treating the subject with a blood glucose control treatment.

### (Item 18)

The method according to any one of the items, in which the HDAC regulator inhibits at least one of class I, class II, and class IV HDACs.

### (Item 19)

The method according to any one of the items, in which the HDAC regulator includes at least one selected from the group consisting of fingolimod (FTY720), givinostat, vorinostat (suberoylanilide hydroxamic acid, SAHA), belinostat, panobinostat, resminostat, abexinostat, mocetinostat, trichostatin A (TSA), romidepsin, apicidin, chidamide, entinostat, tacedinaline, tucidinostat, pracinostat (SB939), quisinostat, tefinostat, rocilinostat, fimepinostat, nanatinostat, domatinostat, trapoxin (TPX), cyclic hydroxamic acid-containing peptide 1 (CHAP1), valproate (VPA), sodium butyrate (NaBu), 4-phenylbutyric acid, OBP-801, ME-344, CG200745, MPT0E014, RGFP966 (MCE, HY-13909), SK-7041, scriptaid, MC1568, MHY2256, ricolinostat (ACY-1215), Tubacin (S2239), Tubathian A, Tubastatin A, Pojamide, AN7, CUDC-101, KD5170, R36465, SOU-HDAC42, LBH589, SFN, phenyl-hexyl isothiocyanate, dacinostat (LAQ824), m-carboxycinnamic acid bis-hydroxamide (CBHA), azelaoyl-bis-hydroxamic acid (ABHA) (N,N'-dihydroxynonanediamide), AR-42, suberoyl bis-hydroxamic acid (SBHA), (pivaloyloxy)methyl butyrate (pivanex, AN-9), diallyl trisulfide (DATS), PCI-24781, nicotinamide, tenovin-1, tenovin-6, sirtinol, EX-527, HBI-8000, kevetrin, CHR-3996, 4SC202, WW437, α-ketoamides, heterocyclic ketones, cambinol, I-7ab, C149, depudecin, SEN 196, COMPOUND6J, JGB 1741, JQ1, I-BET 151, BY27, CUDC907, DWP0016, MC1568, W2, RGFP109, tinostamustine (EDO-S101), J22352, M344, ITF-3056, TMP195 (TFMO 2), MI192, Santacruzamate A(CAY10683), ACY-738, BML-281 (CAY10603), LTB2, CKD-506, WK2-16, PCI-34051, ESM-HDAC528, Merck60, (N-hydroxy-7-2-naphthylthio)heptanomide (HNHA), KBH-A42, AGK2, sulforaphane, MS-275, resveratrol, APHA, curcumin, remetinostat, citarinostat, dacinostat, droxinostat, BRD3308, nimbolide, betaine, and chrysophanol.

### (Item 20)

The method according to any one of the items, in which the HDAC regulator includes at least one selected from the group consisting of fingolimod (FTY720), givinostat, trichostatin A, vorinostat, belinostat, panobinostat, resminostat, abexinostat, quisinostat, pracinostat, rocilinostat, nanatinostat, dacinostat, (N-hydroxy-7-2-naphthylthio)heptanomide, valproate, CG200745, scriptaid, KBH-A42, romidepsin, sodium butyrate, 4-phenylbutyric acid, SK-7041, WW437, entinostat, tacedinaline, mocetinostat, (pivaloyloxy)methyl butyrate, trapoxin, α-ketoamides, heterocyclic ketones, diallyl trisulfide, ricolinostat, Tubacin, azelaoyl-bis-hydroxamic acid, and m-carboxycinnamic acid bis-hydroxamide.

### (Item 21)

The method according to any one of the items, in which the control of blood glucose is performed by at least one selected from the group consisting of administration of a hypoglycemic agent, dietary restriction, and nutrient infusion control.

### (Item 22)

The method according to any one of the items, in which the hypoglycemic agent is selected from the group consisting of insulin, an insulin analog, a biguanide, an insulin secretagogue (a sulfonylurea drug (SU drug) or the like), a fast-acting insulin secretagogue (a glinide drug or the like), an incretin-related drug (a DPP-4 inhibitor, a GLP-1 receptor agonist, or the like), a sugar absorption regulator (α-glucosidase inhibitor (α-GI)), an insulin resistance-ameliorating agent (a thiazolidine drug), a urine glucose evacuant (a SGLT2 inhibitor), and a combination of two or more thereof.

### (Item 23)

The method according to any one of the items, in which the control of blood glucose includes controlling fasting blood glucose to less than 126 mg/dL, controlling a blood glucose level at 2 hours of an oral glucose tolerance test to less than 200 mg/dL, or controlling a casual blood glucose level to less than 200 mg/dL.

### (Item 24)

The method according to any one of the items, in which the method is used for treating diabetes mellitus and/or a disease, disorder, and/or symptom associated with diabetes mellitus.

### (Item 25)

The method according to any one of the items, in which the disease, disorder, and/or symptom includes diabetic complications.

### (Item 26)

The method according to any one of the items, in which the disease, disorder, and/or symptom is selected from the group consisting of a neurological disorder, nephropathy, a hepatic disorder, retinopathy, fatty liver, a gastrointestinal disorder, delayed fracture healing, an eating disorder, and a skin disorder.

### (Item 27)

The method according to any one of the items, further including diagnosing the subject as having abnormal hematopoietic stem cells (HSCs) or cells derived therefrom.

### (Item 28)

The method according to any one of the items, in which the abnormal hematopoietic stem cells have at least one of variable levels of HDAC and increased levels of CD 106.

### (Item 29)

A use of a histone deacetylase (HDAC) regulator in manufacture of a medicine for a treatment and/or prevention of diabetes mellitus and/or a disease, disorder, and/or symptom associated with diabetes mellitus in a subject, in which the medicine is administered in a controlled blood glucose state of the subject.

### (Item 30)

A use of a histone deacetylase (HDAC) regulator in manufacture of a medicine for a treatment and/or prevention of diabetes mellitus and/or a disease, disorder, and/or symptom associated with diabetes mellitus in a subject, in which the medicine is administered in combination with a blood glucose control treatment.

### (Item 31)

The use according to any one of the items, in which the HDAC regulator inhibits at least one of class I, class II, and class IV HDACs.

### (Item 32)

The use according to any one of the items, in which the HDAC regulator includes at least one selected from the group consisting of fingolimod (FTY720), givinostat, vorinostat (suberoylanilide hydroxamic acid, SAHA), belinostat, panobinostat, resminostat, abexinostat, mocetinostat, trichostatin A (TSA), romidepsin, apicidin, chidamide, entinostat, tacedinaline, tucidinostat, pracinostat (SB939), quisinostat, tefinostat, rocilinostat, fimepinostat, nanatinostat, domatinostat, trapoxin (TPX), cyclic hydroxamic acid-containing peptide 1 (CHAP1), valproate (VPA), sodium butyrate (NaBu), 4-phenylbutyric acid, OBP-801, ME-344, CG200745, MPT0E014, RGFP966 (MCE, HY-13909), SK-7041, scriptaid, MC1568, MHY2256, ricolinostat (ACY-1215), Tubacin (S2239), Tubathian A, Tubastatin A, Pojamide, AN7, CUDC-101, KD5170, R36465, SOU-HDAC42, LBH589, SFN, phenyl-hexyl isothiocyanate, dacinostat (LAQ824), m-carboxycinnamic acid bis-hydroxamide (CBHA), azelaoyl-bis-hydroxamic acid (ABHA) (N,N'-dihydroxynonanediamide), AR-42, suberoyl bis-hydroxamic acid (SBHA), (pivaloyloxy)methyl butyrate (pivanex, AN-9), diallyl trisulfide (DATS), PCI-24781, nicotinamide, tenovin-1, tenovin-6, sirtinol, EX-527, HBI-8000, kevetrin, CHR-3996, 4SC202, WW437, α-ketoamides, heterocyclic ketones, cambinol, I-7ab, C149, depudecin, SEN 196, COMPOUND6J, JGB1741, JQ1, I-BET151, BY27, CUDC907, DWP0016, MC1568, W2, RGFP109, tinostamustine (EDO-S101), J22352, M344, ITF-3056, TMP195 (TFMO 2), MI192, Santacruzamate A(CAY10683), ACY-738, BML-281 (CAY10603), LTB2, CKD-506, WK2-16, PCI-34051, ESM-HDAC528, Merck60, (N-hydroxy-7-2-naphthylthio)heptanomide (HNHA), KBH-A42, AGK2, sulforaphane, MS-275, resveratrol, APHA, curcumin, remetinostat, citarinostat, dacinostat, droxinostat, BRD3308, nimbolide, betaine, and chrysophanol.

### (Item 33)

The use according to any one of the items, in which the HDAC regulator includes at least one selected from the group consisting of fingolimod (FTY720), givinostat, trichostatin A, vorinostat, belinostat, panobinostat, resminostat, abexinostat, quisinostat, pracinostat, rocilinostat, nanatinostat, dacinostat, (N-hydroxy-7-2-naphthylthio)heptanomide, valproate, CG200745, scriptaid, KBH-A42, romidepsin, sodium butyrate, 4-phenylbutyric acid, SK-7041, WW437, entinostat, tacedinaline, mocetinostat, (pivaloyloxy)methyl butyrate, trapoxin, α-ketoamides, heterocyclic ketones, diallyl trisulfide, ricolinostat, Tubacin, azelaoyl-bis-hydroxamic acid, and m-carboxycinnamic acid bis-hydroxamide.

### (Item 34)

The use according to any one of the items, in which the control of blood glucose is performed by at least one selected from the group consisting of administration of a hypoglycemic agent, dietary restriction, and nutrient infusion control.

### (Item 35)

The use according to any one of the items, in which the hypoglycemic agent is selected from the group consisting of insulin, an insulin analog, a biguanide, an insulin secretagogue (a sulfonylurea drug (SU drug) or the like), a fast-acting insulin secretagogue (a glinide drug or the like), an incretin-related drug (a DPP-4 inhibitor, a GLP-1 receptor agonist, or the like), a sugar absorption regulator (α-glucosidase inhibitor (α-GI)), an insulin resistance-ameliorating agent (a thiazolidine drug), a urine glucose evacuant (a SGLT2 inhibitor), and a combination of two or more thereof.

### (Item 36)

The use according to any one of the items, in which the control of blood glucose includes controlling fasting blood glucose to less than 126 mg/dL, controlling a blood glucose level at 2 hours of an oral glucose tolerance test to less than 200 mg/dL, or controlling a casual blood glucose level to less than 200 mg/dL.

### (Item 37)

The use according to any one of the items, in which the use is for treating diabetes mellitus and/or a disease, disorder, and/or symptom associated with diabetes mellitus.

### (Item 38)

The use according to any one of the items, in which the disease, disorder, and/or symptom includes diabetic complications.

### (Item 39)

The use according to any one of the items, in which the disease, disorder, and/or symptom is selected from the group consisting of a neurological disorder, nephropathy, a hepatic disorder, retinopathy, fatty liver, a gastrointestinal disorder, delayed fracture healing, an eating disorder, and a skin disorder.

### (Item 40)

The use according to any one of the items, in which the subject is diagnosed as having abnormal hematopoietic stem cells (HSCs) or cells derived therefrom.

### (Item 41)

The use according to any one of the items, in which the abnormal hematopoietic stem cells have at least one of variable levels of HDAC and increased levels of CD106.

### (Item 42)

A histone deacetylase (HDAC) regulator for a treatment and/or prevention of diabetes mellitus and/or a disease, disorder, and/or symptom associated with diabetes mellitus in a subject, in which the HDAC regulator is administered in a controlled blood glucose state of the subject.

### (Item 43)

A histone deacetylase (HDAC) regulator for a treatment and/or prevention of diabetes mellitus and/or a disease, disorder, and/or symptom associated with diabetes mellitus in a subject, in which the HDAC regulator is administered in combination with a blood glucose control treatment.

### (Item 44)

The HDAC regulator according to any one of the items, in which the HDAC regulator inhibits at least one of class I, class II, and class IV HDACs.

### (Item 45)

The HDAC regulator according to any one of the items, in which the HDAC regulator includes at least one selected from the group consisting of fingolimod (FTY720), givinostat, vorinostat (suberoylanilide hydroxamic acid, SAHA), belinostat, panobinostat, resminostat, abexinostat, mocetinostat, trichostatin A (TSA), romidepsin, apicidin, chidamide, entinostat, tacedinaline, tucidinostat, pracinostat (SB939), quisinostat, tefinostat, rocilinostat, fimepinostat, nanatinostat, domatinostat, trapoxin (TPX), cyclic hydroxamic acid-containing peptide 1 (CHAP1), valproate (VPA), sodium butyrate (NaBu), 4-phenylbutyric acid, OBP-801, ME-344, CG200745, MPT0E014, RGFP966 (MCE, HY-13909), SK-7041, scriptaid, MC1568, MHY2256, ricolinostat (ACY-1215), Tubacin (S2239), Tubathian A, Tubastatin A, Pojamide, AN7, CUDC-101, KD5170, R36465, SOU-HDAC42, LBH589, SFN, phenyl-hexyl isothiocyanate, dacinostat (LAQ824), m-carboxycinnamic acid bis-hydroxamide (CBHA), azelaoyl-bis-hydroxamic acid (ABHA) (N,N'-dihydroxynonanediamide), AR-42, suberoyl bis-hydroxamic acid (SBHA), (pivaloyloxy)methyl butyrate (pivanex, AN-9), diallyl trisulfide (DATS), PCI-24781, nicotinamide, tenovin-1, tenovin-6, sirtinol, EX-527, HBI-8000, kevetrin, CHR-3996, 4SC202, WW437, α-ketoamides, heterocyclic ketones, cambinol, I-7ab, C149, depudecin, SEN 196, COMPOUND6J, JGB1741, JQ1, I-BET151, BY27, CUDC907, DWP0016, MC1568, W2, RGFP109, tinostamustine (EDO-S101), J22352, M344, ITF-3056, TMP195 (TFMO 2), MI192, Santacruzamate A(CAY10683), ACY-738, BML-281 (CAY10603), LTB2, CKD-506, WK2-16, PCI-34051, ESM-HDAC528, Merck60, (N-hydroxy-7-2-naphthylthio)heptanomide (HNHA), KBH-A42, AGK2, sulforaphane, MS-275, resveratrol, APHA, curcumin, remetinostat, citarinostat, dacinostat, droxinostat, BRD3308, nimbolide, betaine, and chrysophanol.

### (Item 46)

The HDAC regulator according to any one of the items, in which the HDAC regulator includes at least one selected from the group consisting of fingolimod (FTY720), givinostat, trichostatin A, vorinostat, belinostat, panobinostat, resminostat, abexinostat, quisinostat, pracinostat, rocilinostat, nanatinostat, dacinostat, (N-hydroxy-7-2-naphthylthio)heptanomide, valproate, CG200745, scriptaid, KBH-A42, romidepsin, sodium butyrate, 4-phenylbutyric acid, SK-7041, WW437, entinostat, tacedinaline, mocetinostat, (pivaloyloxy)methyl butyrate, trapoxin, α-ketoamides, heterocyclic ketones, diallyl trisulfide, ricolinostat, Tubacin, azelaoyl-bis-hydroxamic acid, and m-carboxycinnamic acid bis-hydroxamide.

### (Item 47)

The HDAC regulator according to any one of the items, in which the control of blood glucose is performed by at least one selected from the group consisting of administration of a hypoglycemic agent, dietary restriction, and nutrient infusion control.

### (Item 48)

The HDAC regulator according to any one of the items, in which the hypoglycemic agent is selected from the group consisting of insulin, an insulin analog, a biguanide, an insulin secretagogue (a sulfonylurea drug (SU drug) or the like), a fast-acting insulin secretagogue (a glinide drug or the like), an incretin-related drug (a DPP-4 inhibitor, a GLP-1 receptor agonist, or the like), a sugar absorption regulator (α-glucosidase inhibitor (α-GI)), an insulin resistance-ameliorating agent (a thiazolidine drug), a urine glucose evacuant (a SGLT2 inhibitor), and a combination of two or more thereof.

### (Item 49)

The HDAC regulator according to any one of the items, in which the control of blood glucose includes controlling fasting blood glucose to less than 126 mg/dL, controlling a blood glucose level at 2 hours of an oral glucose tolerance test to less than 200 mg/dL, or controlling a casual blood glucose level to less than 200 mg/dL.

### (Item 50)

The HDAC regulator according to any one of the items, in which the HDAC regulator is used for treating diabetes mellitus and/or a disease, disorder, and/or symptom associated with diabetes mellitus.

### (Item 51)

The composition according to any one of the items, in which the disease, disorder, and/or symptom includes diabetic complications.

### (Item 52)

The HDAC regulator according to any one of the items, in which the disease, disorder, and/or symptom is selected from the group consisting of a neurological disorder, nephropathy, a hepatic disorder, retinopathy, fatty liver, a gastrointestinal disorder, delayed fracture healing, an eating disorder, and a skin disorder.

### (Item 53)

The HDAC regulator according to any one of the items, in which the subject is diagnosed as having abnormal hematopoietic stem cells (HSCs) or cells derived therefrom.

### (Item 54)

The HDAC regulator according to any one of the items, in which the abnormal hematopoietic stem cells have at least one of variable levels of HDAC and increased levels of CD106.

In the present disclosure, it is intended that the one or a plurality of features can be provided in further combination in addition to the specified combination. Still further embodiments and advantages of the present disclosure will be appreciated by those skilled in the art upon reading and understanding the following detailed description, if necessary. Advantageous Effects of Invention

The present disclosure provides a novel treatment of diabetes mellitus and/or an associated disease thereof. The present disclosure can eliminate the necessity of donors conventionally required for pancreas transplantation, pancreatic islet transplantation, and the like, which are methods for recovering insulin secretion in patients with diabetes mellitus, and can overcome the limitation in the supplying capacity compared to the number of recipients. In addition, the burden on patients such as surgery that recipients undergoing can also be avoided. As such, the treatment of the present disclosure can be much simpler and less burdensome because the treatment may be achieved by drug administration. In addition, excellent prognosis is expected because radical treatment is possible.

### Brief Description of Drawings

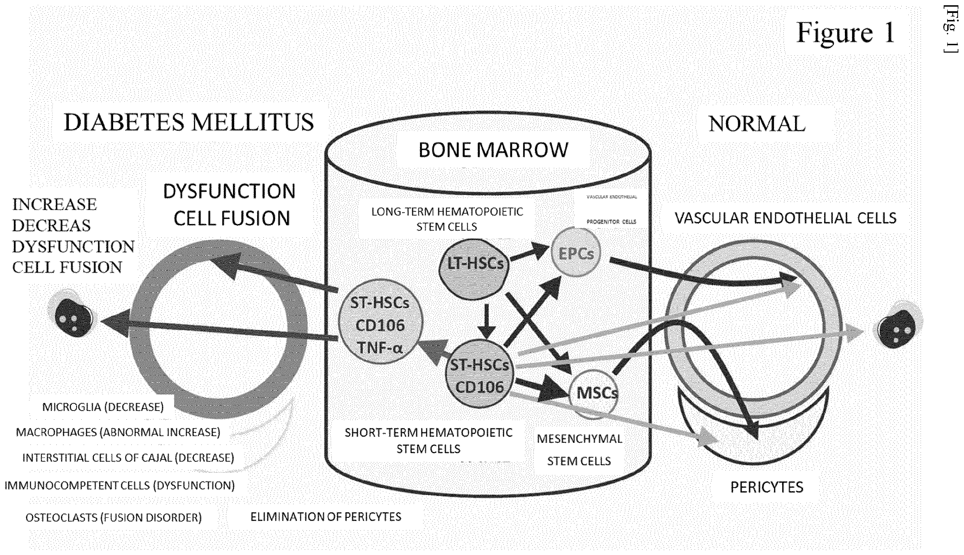

[FIG.1] FIG. 1 illustrates a schematic view of bone marrow cells involved in diabetes mellitus in comparison between diabetes mellitus and normal.

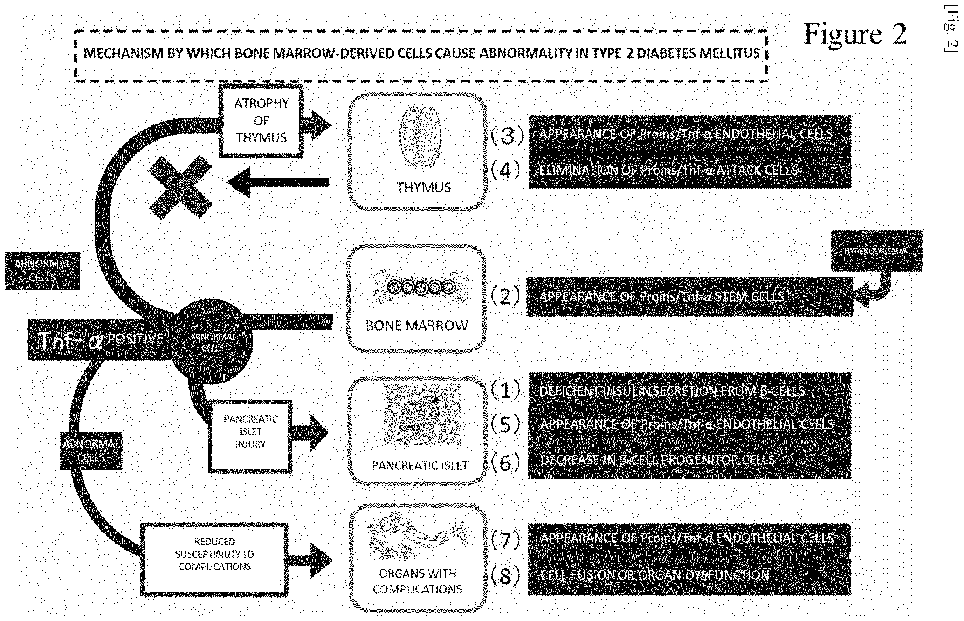

[FIG.2] FIG. 2 is a schematic view illustrating a mechanism by which bone marrow-derived cells cause abnormality in type 2 diabetes mellitus.

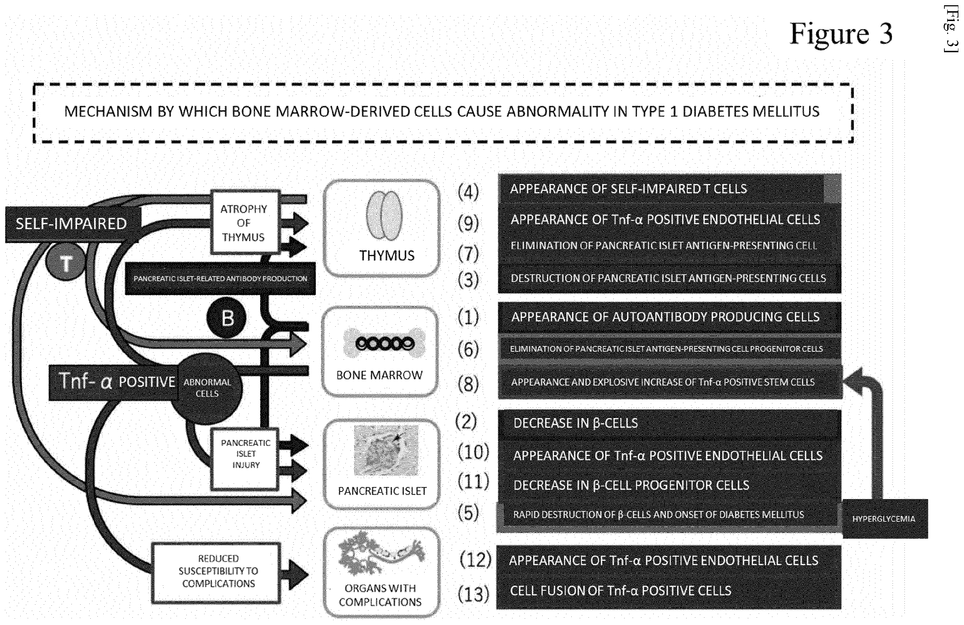

[FIG.3] FIG. 3 is a schematic view illustrating a mechanism by which bone marrow-derived cells cause abnormality in type 1 diabetes mellitus.

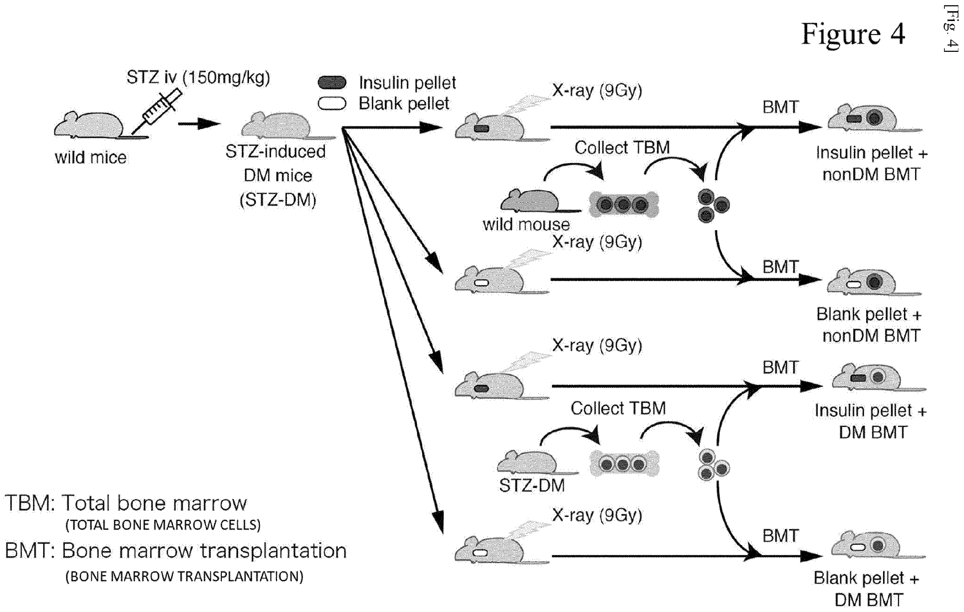

[FIG.4] FIG. 4 is a schematic view illustrating an outline of a bone marrow transplantation treatment of STZ diabetic mice. A summary of transplanting non-diabetes mellitus or diabetes mellitus-derived bone marrow into STZ diabetic mice under an insulin pellet or blank pellet treatment is shown.

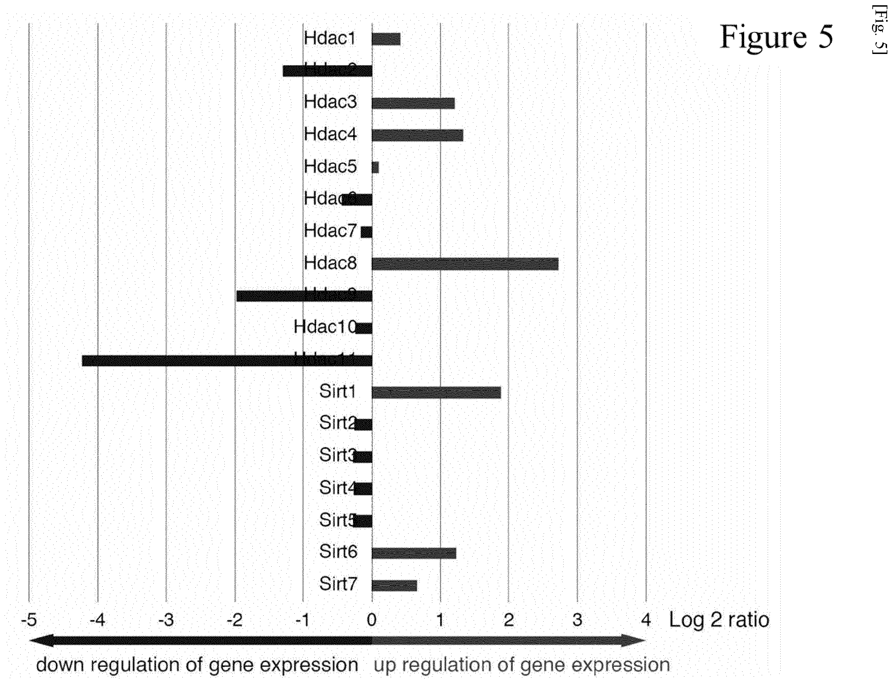

[FIG.5] FIG. 5 illustrates a comparison of mRNA expression of each histone deacetylase gene group (HDACs) in KSL cells of non-DM and STZ-DM mice. The horizontal axis represents mRNA expression magnification (Log2 change) in STZ-DM mice relative to non-DM mice, in which the right shows increased expression and the left shows decreased expression.

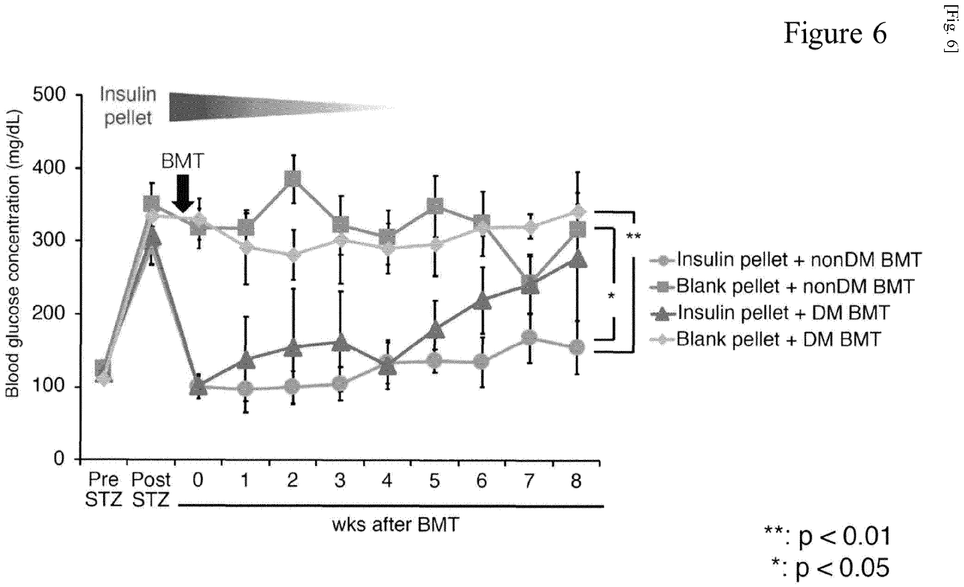

[FIG.6] FIG. 6 illustrates a change in blood glucose level when the treatment illustrated in FIG. 4 is performed. The vertical axis represents the average of the blood glucose level (mg/dL) at each time point together with the standard error. The horizontal axis represents the time course of the treatment in which the time point of bone marrow transplantation is set to week 0. The shelf life of the insulin pellet is also illustrated. *P < 0.05 and **P < 0.01 are illustrated. The results of STZ diabetic mice treated with a blank pellet and diabetic bone marrow transplantation, STZ diabetic mice treated with a blank pellet and non-diabetic bone marrow transplantation, STZ diabetic mice treated with an insulin pellet and diabetic bone marrow transplantation, and STZ diabetic mice treated with an insulin pellet and non-diabetic bone marrow transplantation are shown in descending order of blood glucose levels at week 8.

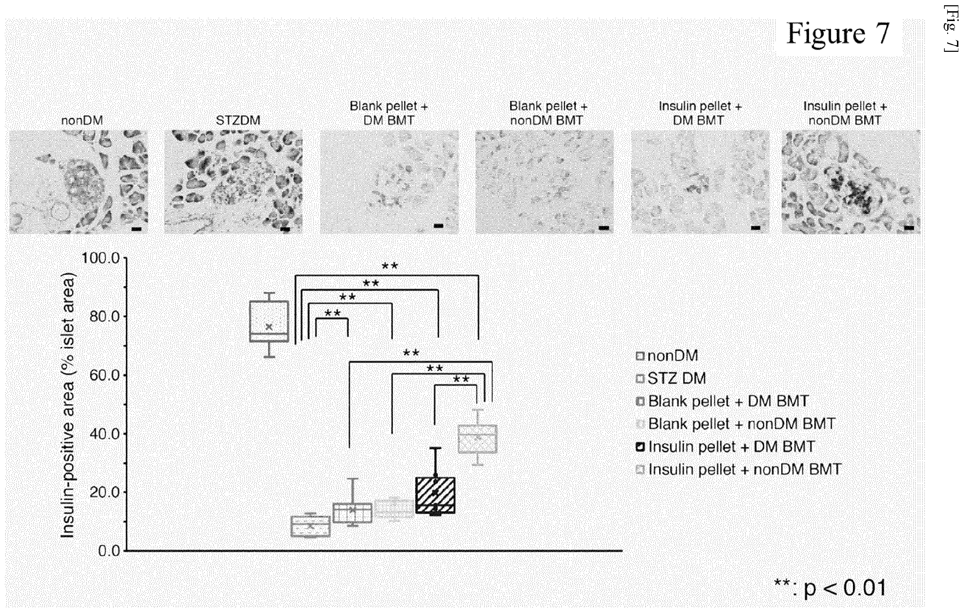

[FIG.7] FIG. 7 illustrates the results of frozen sections of the pancreas after the treatment illustrated in FIG. 4 is performed. (Upper panel) Images of stained insulin and nuclei of frozen sections. Conditions of the mouse from which each section is obtained are displayed on each image. Scale bar indicates 20 µm. (Lower panel) Results of randomly selecting pancreatic islets in each mouse and calculating the percentage (%) of insulin positive cells relative to the size of the pancreatic islets using Image J software. The results of non-diabetic mice, STZ diabetic mice, STZ diabetic mice treated with a blank pellet and diabetic bone marrow transplantation, STZ diabetic mice treated with a blank pellet and non-diabetic bone marrow transplantation, STZ diabetic mice treated with an insulin pellet and diabetic bone marrow transplantation, and STZ diabetic mice treated with an insulin pellet and non-diabetic bone marrow transplantation are shown from the left. **P < 0.01 is illustrated.

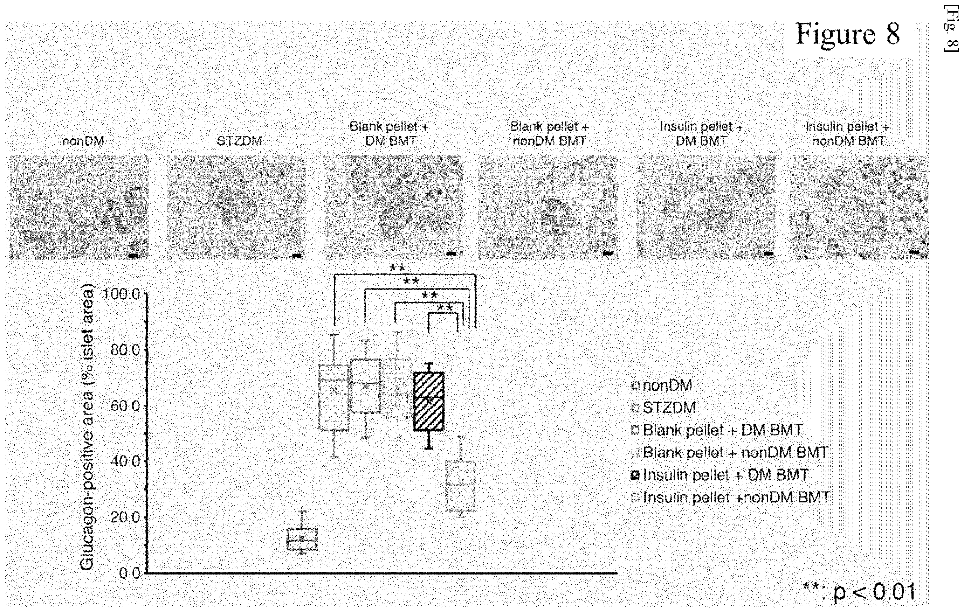

[FIG.8] FIG. 8 illustrates the results of frozen sections of the pancreas after the treatment illustrated in FIG. 4 is performed. (Upper panel) Images of stained glucagon and nuclei of frozen sections. Conditions of the mouse from which each section is obtained are displayed on each image. Scale bar indicates 20 µm. (Lower panel) Results of randomly selecting pancreatic islets in each mouse and calculating the percentage (%) of glucagon positive cells relative to the size of the pancreatic islets using Image J software. The results of non-diabetic mice, STZ diabetic mice, STZ diabetic mice treated with a blank pellet and diabetic bone marrow transplantation, STZ diabetic mice treated with a blank pellet and non-diabetic bone marrow transplantation, STZ diabetic mice treated with an insulin pellet and diabetic bone marrow transplantation, and STZ diabetic mice treated with an insulin pellet and non-diabetic bone marrow transplantation are shown from the left. **P < 0.01 is illustrated.

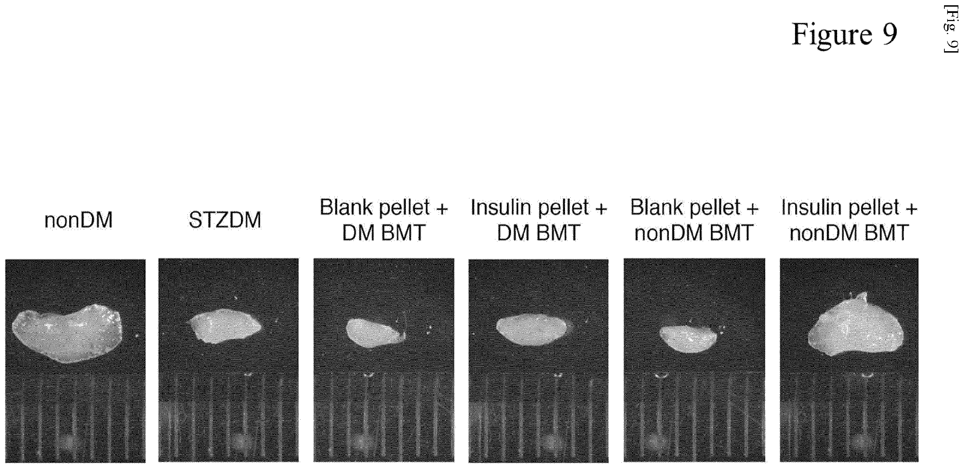

[FIG. 9] FIG. 9 illustrates a comparison of the sizes of typical extracted thymus when the treatment illustrated in FIG. 4 is performed. The thymus extracted from non-diabetic mice, STZ diabetic mice, STZ diabetic mice treated with a blank pellet and diabetic bone marrow transplantation, STZ diabetic mice treated with an insulin pellet and diabetic bone marrow transplantation, STZ diabetic mice treated with a blank pellet and non-diabetic bone marrow transplantation, and STZ diabetic mice treated with an insulin pellet and non-diabetic bone marrow transplantation are shown from the left.

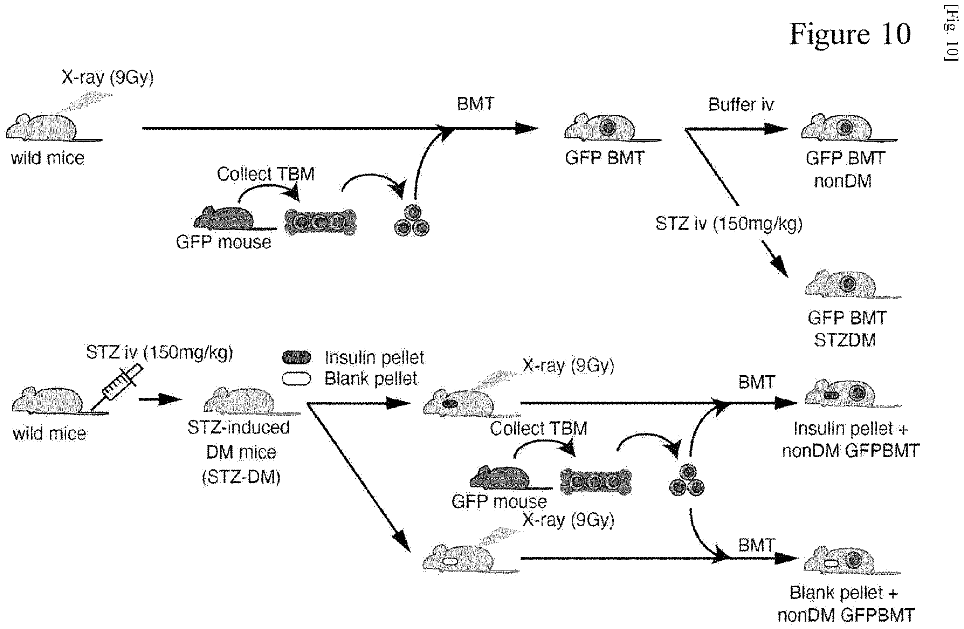

[FIG. 10] FIG. 10 is a schematic view illustrating an outline of a mouse experiment of a combination treatment of bone marrow transplantation using bone marrow cells derived from a transgenic mouse (GFP mouse) into which a green fluorescent protein is genetically introduced and an insulin pellet, and diabetes mellitus induction by STZ.

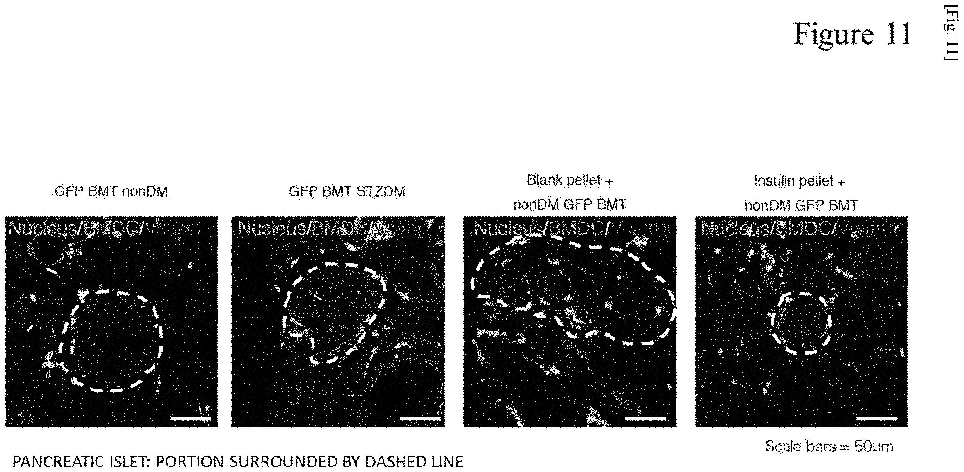

[FIG. 11] FIG. 11 illustrates laser fluorescence microscopic images of pancreatic islets in a mouse transplanted with bone marrow of a GFP mouse. Vcam and nuclei were stained in tissue sections and fluorescence was observed with GFP. Conditions of the mouse from which each section is obtained are displayed on each image. Scale bar indicates 50 µm. Blue represents nuclei (DAPI), green represents bone marrow-derived cells (GFP), and red represents Vcam1 (Tomato). The white dashed line represents a pancreatic islet outer boundary.

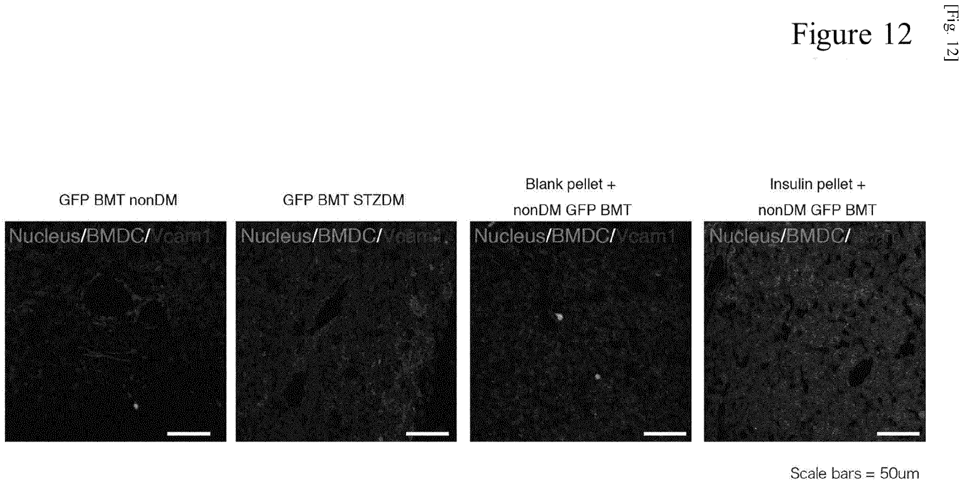

[FIG. 12] FIG. 12 illustrates laser fluorescence microscopic images of thymus in a mouse transplanted with bone marrow of a GFP mouse. Vcam and nuclei were stained in tissue sections and fluorescence was observed with GFP. Conditions of the mouse from which each section is obtained are displayed on each image. Scale bar indicates 50 µm. Blue represents nuclei (DAPI), green represents bone marrow-derived cells (GFP), and red represents Vcam1 (Tomato).

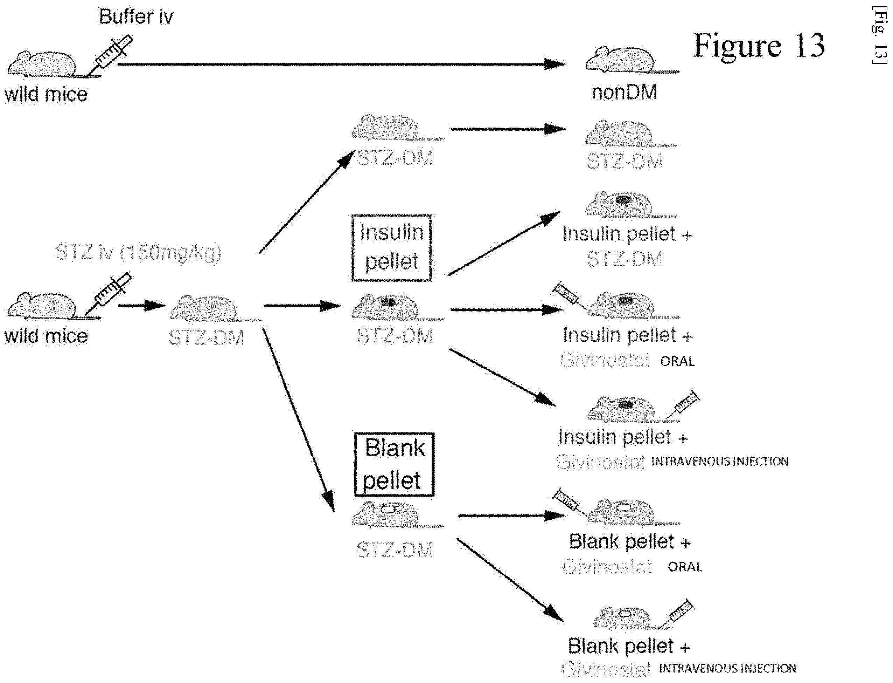

[FIG. 13] FIG. 13 illustrates a schematic of an experiment of Example 3 comparing streptozotocin-induced diabetic mice treated with or without givinostat under an insulin pellet or blank pellet treatment. A test was performed using mice without diabetes mellitus induction by streptozotocin (nonDM, control), and each of streptozotocin-induced diabetic mice with a blank pellet + no givinostat treatment (STZDM), an insulin pellet + no givinostat treatment, a blank pellet + a givinostat treatment (oral or intravenous), and an insulin pellet + a givinostat treatment (oral or intravenous).

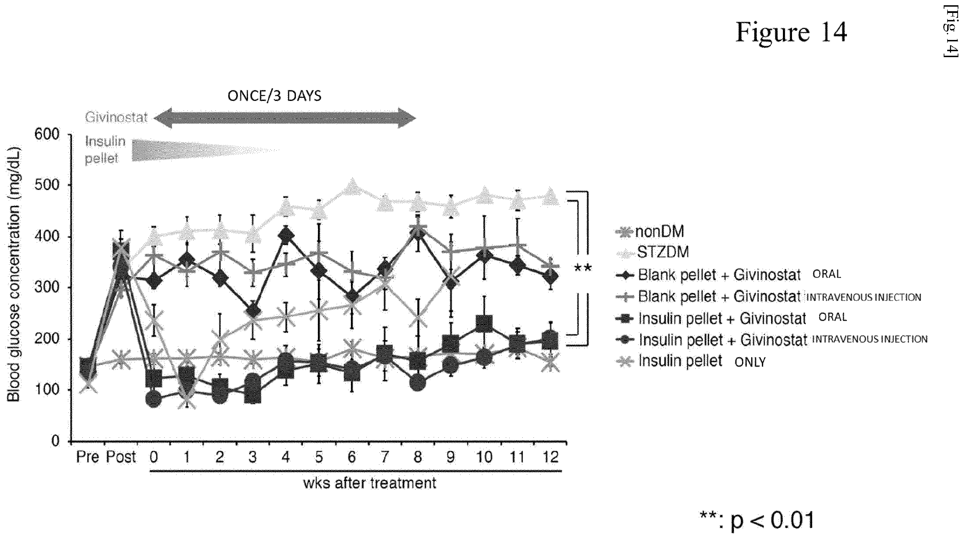

[FIG. 14] FIG. 14 illustrates a change in blood glucose level of each mouse in the experiment of Example 3. The vertical axis represents the average of the blood glucose level (mg/dL) at each time point together with the standard error. The horizontal axis represents the time course of the treatment (weeks) in which the time point at which the givinostat treatment is started is set to week 0. Each mark represents a correspondence between the polygonal line and the type of the mouse.

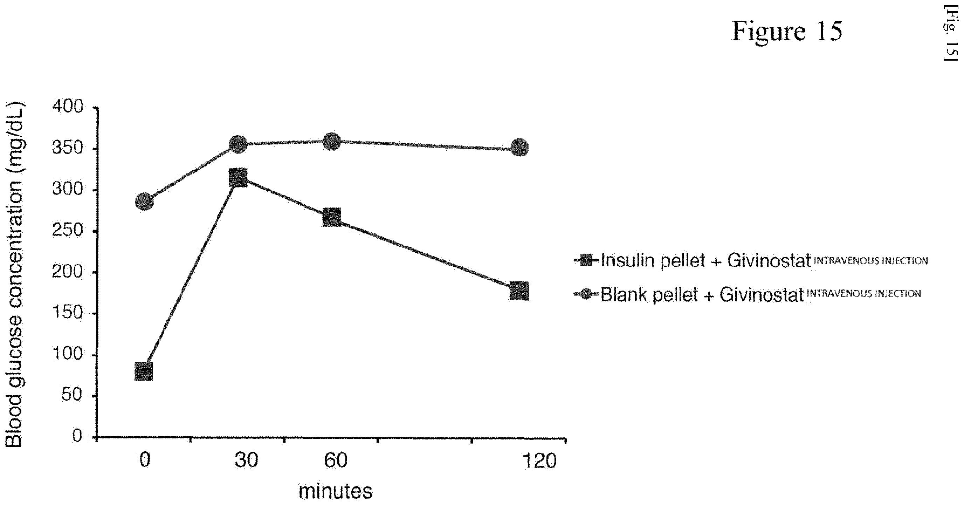

[FIG. 15] FIG. 15 illustrates blood glucose levels after an intraperitoneal glucose tolerance test (IPGTT) in each streptozotocin-induced diabetic mouse with a blank pellet + a givinostat treatment and with an insulin pellet + a givinostat treatment in the experiment of Example 3. The vertical axis represents the average of the blood glucose level (mg/dL) at each time point, and the horizontal axis represents the time course after glucose injection.

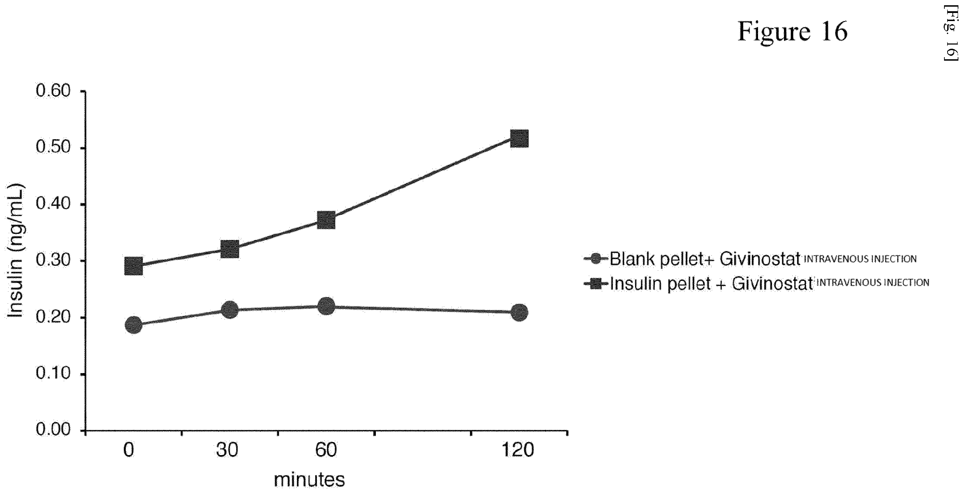

[FIG. 16] FIG. 16 illustrates insulin in blood after an intraperitoneal glucose tolerance test (IPGTT) in each streptozotocin-induced diabetic mouse with a blank pellet + a givinostat treatment and with an insulin pellet + a givinostat treatment in the experiment of Example 3. The vertical axis represents the average of the insulin in blood (ng/mL) at each time point, and the horizontal axis represents the time course after glucose injection.

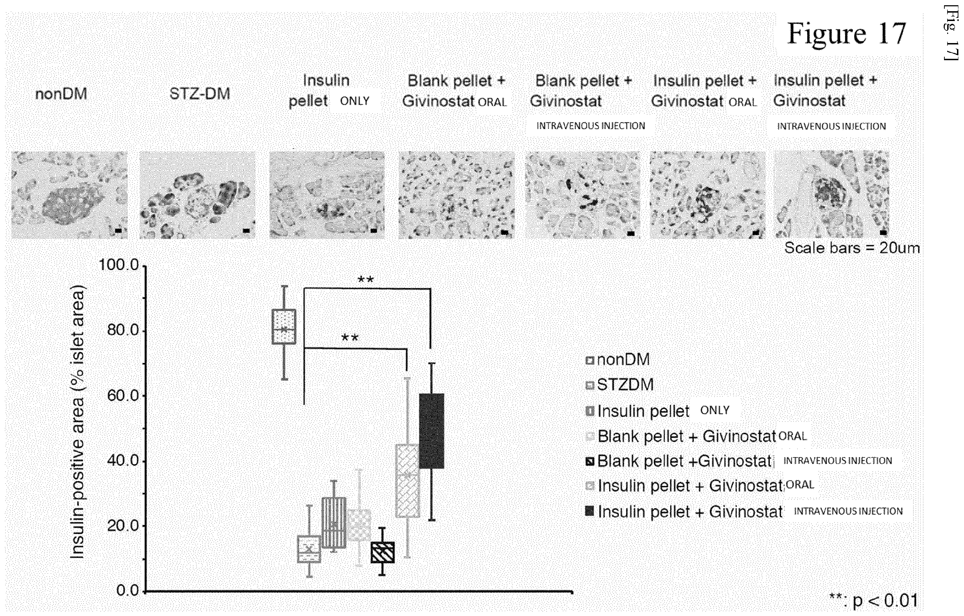

[FIG. 17] FIG. 17 illustrates the observation results of insulin immunohistochemical staining of frozen sections of pancreas when the treatment of the experiment of Example 3 is performed. (Upper panel) Images of stained insulin and nuclei of frozen sections. Scale bar indicates 20 µm. (Lower panel) Results of randomly selecting pancreatic islets in each mouse and calculating the percentage (%) of insulin positive cells relative to the size of the pancreatic islets using Image J software. Mice without diabetes mellitus induction by streptozotocin (control), and each of streptozotocin-induced diabetic mice with a blank pellet + no givinostat treatment, a blank pellet + no givinostat treatment, a blank pellet + a givinostat treatment (oral), an insulin pellet + a givinostat treatment (intravenous), an insulin pellet + a givinostat treatment (oral), and an insulin pellet + a givinostat treatment (intravenous) are shown from the left. **P < 0.01 is illustrated.

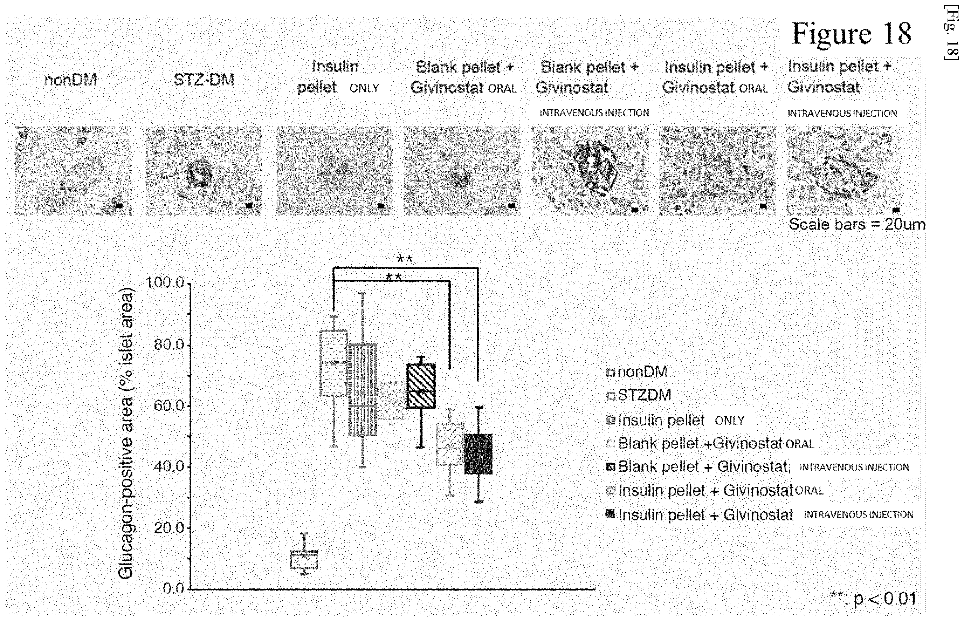

[FIG. 18] FIG. 18 illustrates the observation results of glucagon immunohistochemical staining of frozen sections of pancreas when the treatment of the experiment of Example 3 is performed. (Upper panel) Images of stained glucagon and nuclei of frozen sections. Scale bar indicates 20 µm. (Lower panel) Results of randomly selecting pancreatic islets in each mouse and calculating the percentage (%) of glucagon positive cells relative to the size of the pancreatic islets using Image J software. Mice without diabetes mellitus induction by streptozotocin (control), and each of streptozotocin-induced diabetic mice with a blank pellet + no givinostat treatment, a blank pellet + no givinostat treatment, a blank pellet + a givinostat treatment (oral), an insulin pellet + a givinostat treatment (intravenous), an insulin pellet + a givinostat treatment (oral), and an insulin pellet + a givinostat treatment (intravenous) are shown from the left. **P < 0.01 is illustrated.

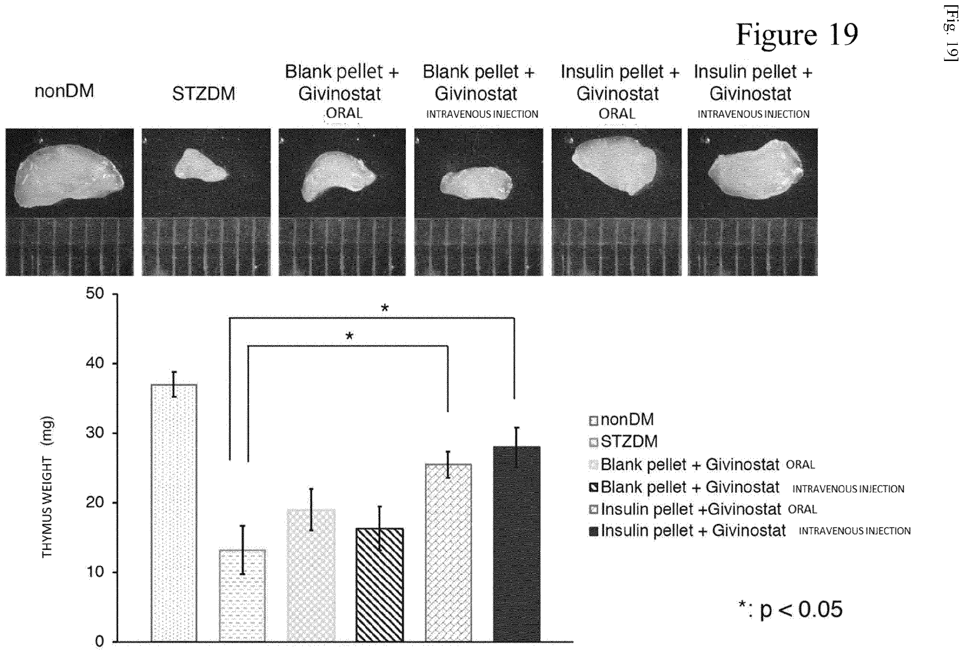

[FIG. 19] FIG. 19 illustrates the size (upper photograph) and weight (lower graph) of the extracted thymus of each mouse at the end of the test period of the experiment of Example 3. The vertical axis of the graph represents the average of thymus weights (mg) with a standard error (in a case where n= 2). In both the photograph and the graph, each of streptozotocin-induced diabetic mice with a blank pellet + no givinostat treatment, a blank pellet + a givinostat treatment (oral), a blank pellet + a givinostat treatment (intravenous), an insulin pellet + a givinostat treatment (oral), and an insulin pellet + a givinostat treatment (intravenous), and mice without diabetes mellitus induction by streptozotocin (control) are shown from the left. *P < 0.05 is illustrated.

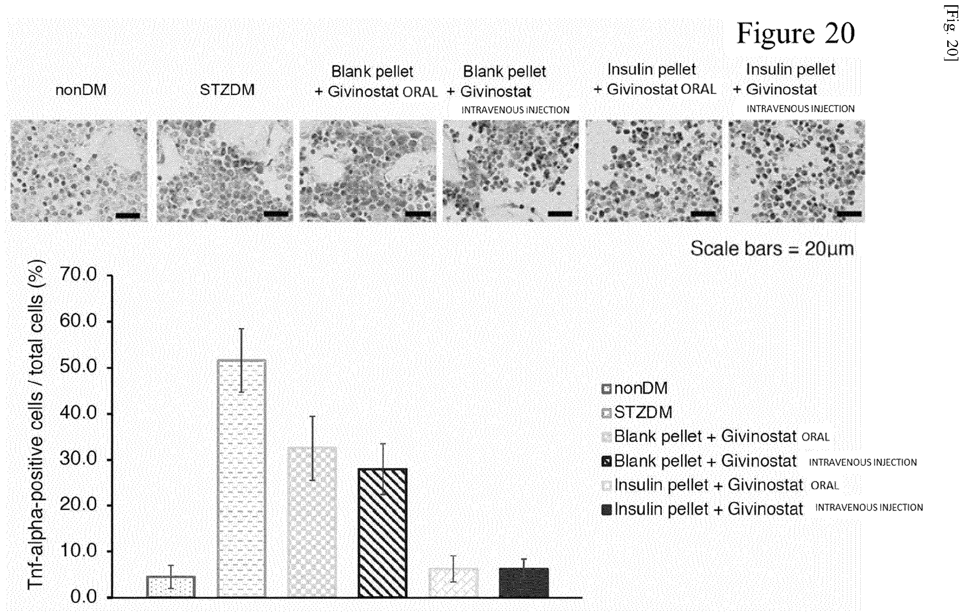

[FIG.20] FIG. 20 illustrates the observation results of TNF-α immunohistological staining of demineralized bone tissue sections when the treatment of the experiment of Example 3 is performed. (Upper panel) Images of stained TNF-α (Ni-DAB staining, brown) and nuclei (hematoxylin staining, purple) of demineralized bone tissue sections. Scale bar indicates 20 µm. (Lower panel) Results of randomly selecting bone marrow in each mouse and calculating the percentage (%) of TNF-α positive cells among cells with stained nuclei (purple) using Image J software. Mice without diabetes mellitus induction (control), and each of streptozotocin-induced diabetic mice with a blank pellet + no givinostat treatment, a blank pellet + a givinostat treatment (oral), a blank pellet + a givinostat treatment (intravenous), an insulin pellet + a givinostat treatment (oral), and an insulin pellet + a givinostat treatment (intravenous) are shown from the left.

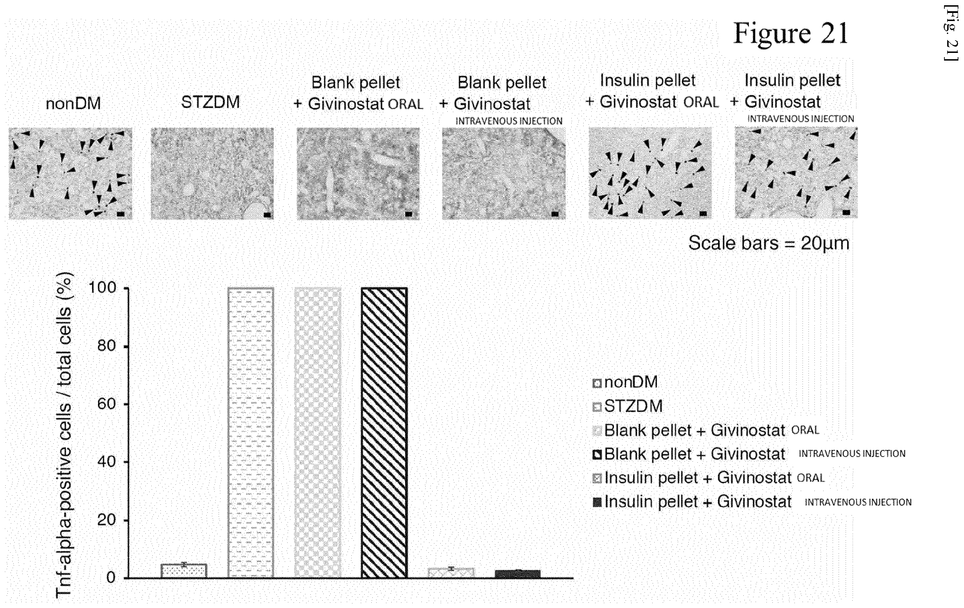

[FIG.21] FIG. 21 illustrates the observation results of TNF-α immunohistological staining of thymus when the treatment of the experiment of Example 3 is performed. (Upper panel) Images of stained TNF-α (Ni-DAB staining, brown, indicated by the black arrow) and nuclei (hematoxylin staining, purple) of thymus. Scale bar indicates 20 µm. (Lower panel) Results of randomly selecting thymus tissue in each mouse and calculating the percentage (%) of TNF-α positive cells among cells with stained nuclei (purple) using Image J software. Mice without diabetes mellitus induction (control), and each of streptozotocin-induced diabetic mice with a blank pellet + no givinostat treatment, a blank pellet + a givinostat treatment (oral), a blank pellet + a givinostat treatment (intravenous), an insulin pellet + a givinostat treatment (oral), and an insulin pellet + a givinostat treatment (intravenous) are shown from the left.

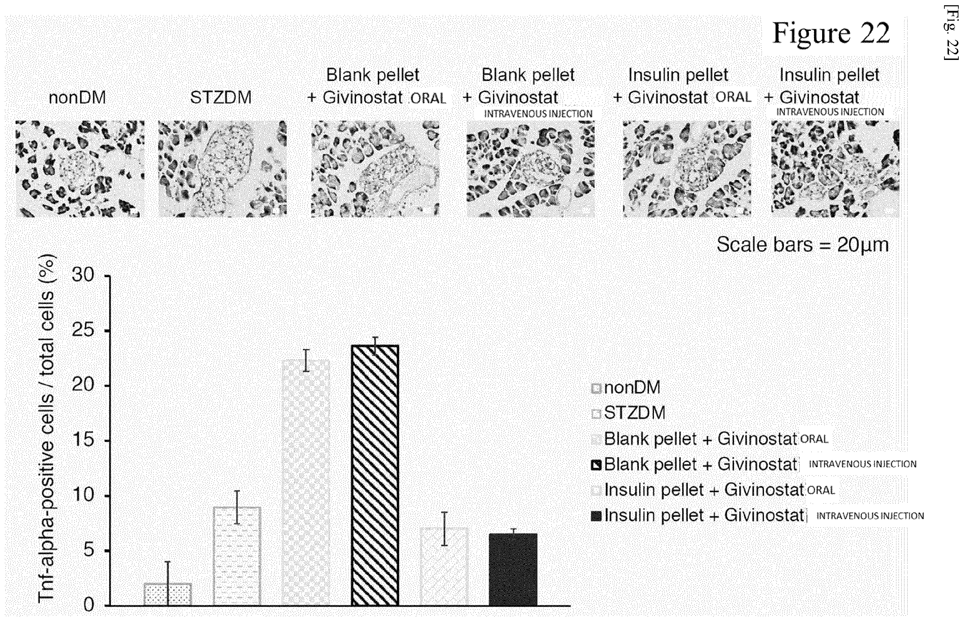

[FIG.22] FIG. 22 illustrates the observation results of TNF-α immunohistological staining of pancreatic sections when the treatment of the experiment of Example 3 is performed. (Upper panel) Images of stained TNF-α (Ni-DAB staining, brown) and nuclei (hematoxylin staining, purple) of pancreatic sections. Scale bar indicates 20 µm. (Lower panel) Results of randomly selecting pancreatic islets in the sections in each mouse and calculating the percentage (%) of TNF-α positive cells among cells with stained nuclei (purple) using Image J software. Mice without diabetes mellitus induction (control), and each of streptozotocin-induced diabetic mice with a blank pellet + no givinostat treatment, a blank pellet + a givinostat treatment (oral), a blank pellet + a givinostat treatment (intravenous), an insulin pellet + a givinostat treatment (oral), and an insulin pellet + a givinostat treatment (intravenous) are shown from the left.

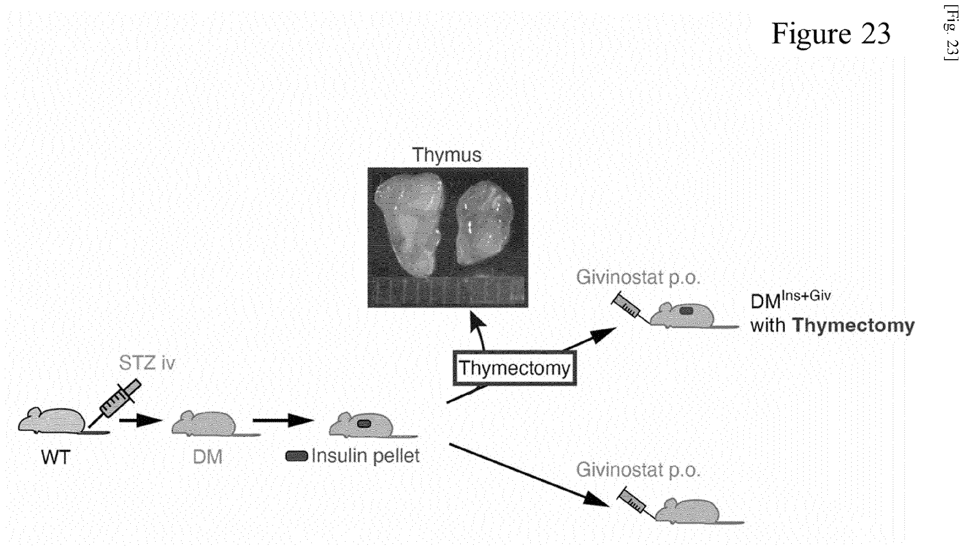

[FIG.23] FIG. 23 is a schematic view illustrating an outline of a treatment of thymectomized STZ diabetic mice in Example 8.

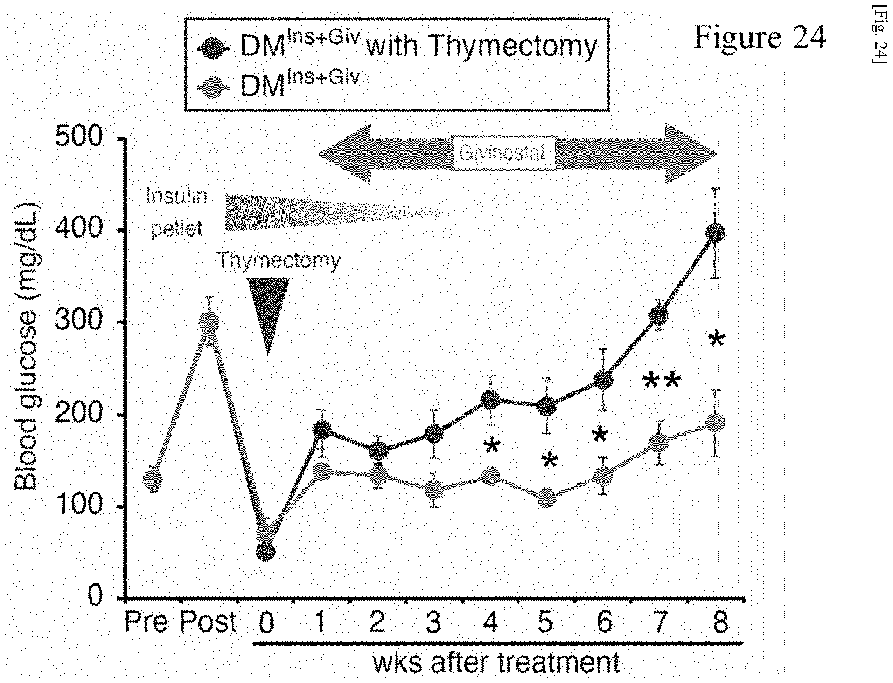

[FIG.24] FIG. 24 illustrates a change in blood glucose level when the treatment illustrated in FIG. 23 is performed. The vertical axis represents the average of the blood glucose level (mg/dL) at each time point together with the standard error. The horizontal axis represents the time course of the treatment in which the time point of the thymectomy treatment is set to week 0. The shelf life of the insulin pellet is also illustrated. *P < 0.05 and **P < 0.01 are illustrated. A higher blood glucose level at week 8 is a result of the thymectomy group.

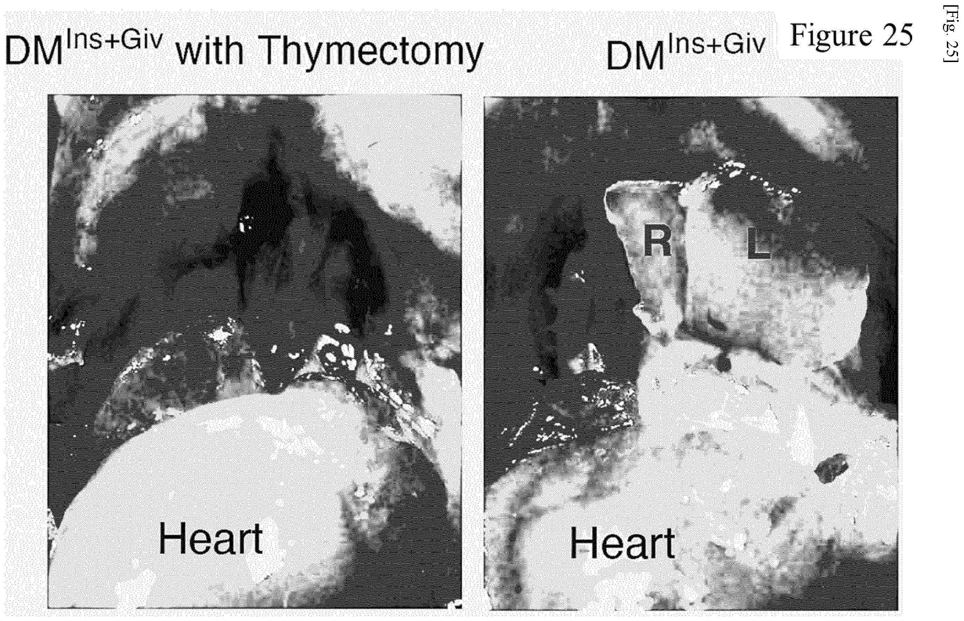

[FIG.25] FIG. 25 illustrates photographs of the periphery of the thymus at week 8 of mice in a thymectomy group (left) and a control group (right) that are subjected to the treatment illustrated in FIG. 23.

### Mode for Carrying Out the Invention

Hereinafter, the present disclosure will be described while showing the best mode. Throughout the present specification, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used in the present specification are to be understood as being used in the sense commonly used in the art unless otherwise stated. Therefore, unless defined otherwise, all technical and scientific terms used in the present specification have the same meaning as commonly understood by those skilled in the art to which the present disclosure pertains. In the case of conflict, the present specification (including definitions) will control.

Hereinafter, the definitions of the terms and/or basic technical concepts that are particularly used in the present specification will be described as appropriate.

### (Definitions)

As used herein, "diabetes mellitus" is used in the meaning that is commonly used in the art, and is generally classified into type 1 diabetes mellitus caused by depletion of insulin due to destruction of pancreatic β-cells and type 2 diabetes mellitus caused by a decrease in the ability of glucose uptake into the muscle and fat tissue resulting from a reduction in the amount of insulin secreted from β-cells of islets of Langerhans (pancreatic islets) of the pancreas due to obesity or the like, except for diabetes mellitus associated with pregnancy and diabetes mellitus due to specific genetic abnormalities. Note that, according to the results of the present disclosure, it is presumed that both type 1 diabetes mellitus and type 2 diabetes mellitus are caused by a common cellular cause mediated by abnormalities in hematopoietic stem cells in the bone marrow due to sustained hyperglycemia and immune abnormalities derived therefrom, which is completely different from what has been considered to be the cause. Diabetes mellitus can be diagnosed by, for example, fasting blood glucose ≥ 126 mg/dL and HbA1c ≥ 6.5% in two or more tests, a 2-hour value of 200 mg/dL or more in an oral glucose tolerance test (75 g OGTT), or a casual blood glucose level of 200 mg/dL or more. As used herein, the term "diabetes mellitus" also includes maturityonset diabetes mellitus of the young and prediabetes mellitus.

As used herein, a "disease associated with diabetes mellitus" or a "disease, disorder, and/or symptom associated with diabetes mellitus" can include any disease, disorder, and symptom associated with diabetes mellitus. Examples thereof include diabetic neuropathy, diabetic retinopathy, diabetic nephropathy, secondary diabetes mellitus, diabetic coma, disturbance of consciousness, stomachache, leg cramp, neuropathy, hyperosmolar hyperglycemic syndrome, albuminuria, edema, renal failure, blindness, Alzheimer's type dementia, myocardial infarction, obstructive arteriosclerosis, cerebral infarction, fatty liver, skin symptoms (such as diabetic lipoid necrobiosis), a decrease in wound healing ability, susceptibility to infections (such as septicemia), cancer (hepatic cancer, renal cancer, pancreatic cancer, colon cancer, gastric cancer, hepatic cancer, ovarian cancer, large intestine cancer, colon cancer, or the like), diabetic ketoacidosis, cardiac disease, cerebral vascular disorder, steroid diabetes mellitus, constipation, dizziness (orthostatic hypotension), erectile dysfunction, myocardial infarction, chest pain, severe appendicitis, peritoneal irritation symptom, low temperature burn, gestational diabetes mellitus, mouth dryness, polydipsia, and polyuria. It is understood that the treatment of diabetes mellitus of the present disclosure enables a fundamental treatment rather than a symptomatic treatment of the symptoms appearing as an associated disease of diabetes mellitus, and is different from the associated diseases of diabetes mellitus known in the related art.

As used herein, the term "non-diabetic subject" means a subject who does not have diabetes mellitus or an associated disease thereof unless otherwise specified.

As used herein, the term "control of blood glucose" or "blood glucose control" refers to both the performance of a treatment that lowers blood glucose (blood glucose concentration: fasting blood glucose, including blood glucose during glucose tolerance test, casual blood glucose, and the like) and the state in which blood glucose is maintained in the therapeutic range described in the present specification. Examples of the treatment for lowering blood glucose include administration of a hypoglycemic agent, dietary restriction, and nutrient infusion control.

Therefore, as used herein, the "state in which blood glucose is controlled" may be achieved by a treatment that actively keeps blood glucose within a certain range, or may be a case where blood glucose is kept within the certain range without doing anything. Alternatively, even when it is not the intended treatment, a case where the blood glucose falls within the certain range also corresponds to the "state in which blood glucose is controlled".

As used herein, the term "treatment" refers to preventing, preferably maintaining of the current condition, more preferably alleviating, and still more preferably curing deterioration of a disease or disorder when the disease or disorder is in such a state, and includes exerting a symptom ameliorating effect or a prophylactic effect on the disease of a patient or one or more symptoms associated with the disease. As used herein, the term "alleviation" refers to reducing the severity of a disease or disorder when it becomes such a condition, and the term "cure" refers to no longer being diagnosed as having such a disease or disorder. Performing diagnosis in advance and performing an appropriate treatment is referred to as a "companion treatment", and a diagnostic drug therefor may be referred to as a "companion diagnostic drug".

As used herein, the term "prevention" refers to preventing a certain disease or disorder from being in such a state prior to being in such a state. The drug of the present disclosure can be used to perform a diagnosis, and if necessary, the drug of the present disclosure can be used to prevent, for example, diabetes mellitus or take measures for prevention.

As used herein, the term "diagnosis" refers to identifying various parameters associated with a condition (for example, a disease or a disorder) or the like in a subject to determine the current or future state of such a condition. The condition in the body can be investigated by using the method, composition, or system of the present disclosure. Such information can be used to select and determine various parameters such as condition in the subject, and a formulation or method for the treatment or prevention to be administered. As used herein, the "diagnosis" when narrowly defined refers to diagnosis of the current state, but when broadly defined, the "diagnosis" includes "early diagnosis", "predictive diagnosis", "prediagnosis", and the like. Since the diagnostic method of the present disclosure in principle can utilize what comes out from a body and can be conducted away from a medical practitioner such as a physician, the present disclosure is industrially useful. As used herein, in order to clarify that the method can be conducted away from a medical practitioner such as a physician, "predictive diagnosis, prediagnosis, or diagnosis" may be particularly referred to as "assisting".

As used herein, the "histone deacetylase (HDAC)" is a family of proteins considered to have an enzymatic activity of releasing lysine acetylation modification on proteins such as histones, and HDAC1 to 11, SIRT1 to 7, and the like are known as members thereof. The enzymatic activity of HDAC1 to 11 is considered to be zinc-ion dependent, HDAC1, 2, 3, and 8 are classified into class I, HDAC4, 5, 7, and 9 are classified into class IIa, HDAC6 and 10 are classified into class IIb, and HDAC11 is classified into class IV. The enzymatic activity of SIRT1 to 7 is considered to be NAD+ dependent, and SIRT1 to 7 are classified into HDAC of class III. Hereinafter, each HDAC and a representative nucleic acid sequence and amino acid sequence thereof are shown below.

**[Table 1]**

| Classification | Name | Representative nucleic acid sequence | Representative amino acid sequence |
|---|---|---|---|
| Class I | HDAC1 | NM_004964.2 | NP_004955.2 |
| | HDAC2 | NM_001527.3 | NP_001518.3 |
| | HDAC3 | NM_003883.3 | NP_003874.2 |
| | HDAC8 | NM_001166418.1 | NP_001159890.1 |
| Class IIa | HDAC4 | NM_006037.3 | NP_006028.2 |
| | HDAC5 | NM_005474.4 | NP_005465.2 |
| | HDAC7 | NM_015401.4 | NP_056216.2 |
| | HDAC9 | NM_001204144.2 | NP_001191073.1 |
| Class IIb | HDAC6 | NM_001321226.1 | NP_001308155.1 |
| | HDAC10 | NM_032019.5 | NP_114408.3 |
| Class IV | HDAC11 | NM_024827.3 | NP_079103.2 |
| Class III | SIRT1 | NM_012238.4 | NP_036370.2 |
| | SIRT2 | NM_012237.3 | NP_036369.2 |
| | SIRT3 | NM_012239.5 | NP_036371.1 |
| | SIRT4 | NM_012240.2 | NP_036372 |
| | SIRT5 | NM_012241.4 | NP_036373.1 |
| | SIRT6 | NM_016539.3 | NP_057623.2 |
| | SIRT7 | NM_016538.2 | NP_057622.1 |

As used herein, the term "regulation" of HDAC or the like means that the function of HDAC or the like is increased, decreased, or eliminated by any means. The "regulation" includes "inhibition" and "activation". As used herein, the term "inhibition" of HDAC or the like means that the function of HDAC or the like is decreased or eliminated by any means. For example, the inhibition of HDAC can result in a decrease or elimination of enzymatic activity of HDAC, a decrease or elimination of transcription of HDAC gene, a decrease or elimination of translation of HDAC protein, promotion of degradation of HDAC protein, and the like. As used herein, the term "activation" of HDAC or the like means to increase the function of HDAC or the like by any means, and may be, for example, to increase the enzymatic activity of HDAC, increase the transcription of HDAC gene, increase the translation of HDAC protein, suppress the degradation of HDAC protein, or the like. As used herein, the term "HDAC regulator" means a drug that regulates HDAC by any means, and includes an "HDAC inhibitor" and an "HDAC activator". As used herein, the term "HDAC inhibitor" means a drug that inhibits HDAC by any means, and the term "HDAC activator" means a drug that activates HDAC by any means.

As used herein, the term "hematopoietic stem cell" or "HSC" refers to a stem cell capable of differentiating into a blood cell line. In a human adult, a hematopoietic stem cell is mainly present in the bone marrow and gives rise to a white blood cell (neutrophil, eosinophil, basophil, lymphocyte, monocyte, or macrophage), a red blood cell, a platelet, a mast cell, and a dendritic cell. A human hematopoietic stem cell can be characterized by, for example, being CD34 positive and Thy-1 positive, and also can be characterized by being Lineage negative, CD34 positive, CD38 negative, CD90 positive, and CD45RA negative (that is, Lin⁻CD34⁺CD38⁻CD90⁺CD45RA⁻) (for example, see Proc Natl Acad Sci USA. 2011 Dec. 13; 108(50):20012-20017). A murine hematopoietic stem cell can be characterized as a c-kit positive, Sca-1 positive, and lineage marker negative (KSL) cell. In addition, a hematopoietic stem cell also can be characterized by being negative for both of two types of optical filters, Hoechst blue and Hoechst red, when a bone marrow cell stained with Hoechest 33342 dye is excited with ultraviolet light (350 nm) and developed with these optical filters. A hematopoietic stem cell can be subclassified into a long-term hematopoietic stem cell (LT-HSC), a short-term hematopoietic stem cell (ST-HSC), and the like. For example, Cytometry Research 19(2):25-32, 2009 can be referred to for the features of a hematopoietic stem cell.

As used herein, the term "long-term hematopoietic stem cell (LT-HSC)" refers to a hematopoietic stem cell having a long-term bone marrow cell re-establishing ability. LT-HSC is generally identified with a surface antigen marker and can be expressed as CD34 negative, CD150 positive, CD48 negative, Lin negative, seal positive, and c-kit positive (or Lineage⁻c-Kit⁺Sca-1⁺CD34^{-/low}CD150⁺ cell) (for example, in a mouse). In addition, LT-HSC also can be expressed as CD34 negative and CD38 negative (or CD34⁻CD38⁻ cell) (for example, in a human) (for example, Nat Immunol. 2010 July; 11(7):585-93; Blood 108, 2446-2454, 2006).

As used herein, the term "short-term hematopoietic stem cell (ST-HSC)" refers to a hematopoietic stem cell having a short-term bone marrow re-establishing ability. ST-HSC is generally identified with a surface antigen marker and can be expressed as CD34 positive, CD150 positive, CD48 negative, Lin negative, sca-1 positive, and c-kit positive (for example, in a mouse). In addition, ST-HSC also can be expressed as CD34 positive and CD38 negative (or CD34⁺CD38⁻ cell) (for example, in a human) (for example, Nat Immunol. 2010 July; 11(7):585-93; Blood 108, 2446-2454, 2006).

As used herein, the term "abnormal hematopoietic stem cell" or "abnormal HSC" refers to a hematopoietic stem cell that expresses an abnormal function and/or lacks at least a part of a normal function. Typically, an abnormal HSC refers to a cell in which a gene or protein of HDAC, TNF-α, CD 106 or a functional equivalent thereof is expressed and/or functions at a level different from normal levels (reduced, increased, or eliminated).

As used herein, a gene or protein being "not expressed at a normal level" refers to the gene or protein being expressed in an amount or level that is not the expression level found in cells having a normal function. For example, regarding CD106, the case where CD106 deviates from the normal expression (normal value observed in a normal cell) can be determined to be abnormal. Preferably, the case where CD106 is expressed more than normal can be determined to be abnormal. For example, KSL cells are fractionated with FACS from non-diabetic and diabetic mice, RNA is extracted, and then the gene expression level is subjected to comparative analysis using QT-PCR, such that it is possible to test expression (for example, overexpression) at an abnormal level of the gene. For example, protein expression at a level that is not normal can be tested by performing FACS analysis, detecting a surface antigen of an abnormal hematopoietic stem cell, and comparing a diabetic subject and a non-diabetic subject.

As used herein, a gene or protein "not functioning at a normal level" refers to a function at a level that is observed in a cell having a normal function that is not observed in the gene or protein. For example, regarding CD106, the case where CD106 deviates from a level at which CD106 normally functions (normal level or value observed in a normal cell) can be determined to be abnormal. Preferably, the case where the function of CD106 is observed at a higher level than normal can be determined to be abnormal. For example, a protein not functioning at a normal level can be tested by performing FACS analysis, detecting a surface antigen of an abnormal hematopoietic stem cell, and comparing a diabetic subject and a non-diabetic subject to observe activation of the protein.

As used herein, "CD 106" is one type of surface antigen and is a type I membrane protein of a member of the Ig superfamily, which is also known as adhesion molecule VCAM-1 (Vascular cell adhesion molecule-1) or INCAM-100. CD106 is a cell surface sialoglycoprotein expressed by a cytokine-activated endothelium and is known to mediate white blood cell-endothelial cell adhesion and signaling. Representative examples in humans include VCAM-1 isoform a precursor (nucleic acid sequence: NM_001078.4, amino acid sequence: NP_001069.1), VCAM-1 isoform b precursor (nucleic acid sequence: NM_080682.2, amino acid sequence: NP_542413.1), and VCAM-1 isoform c precursor (nucleic acid sequence: NM_001199834.1, amino acid sequence: NP_001186763.1).

As used herein, "CD34" is a surface protein which is considered to be involved in attachment of a stem cell to a bone marrow extracellular matrix or a stromal cell. CD34 is known to be highly glycosylated, and phosphorylated by protein kinase C. Representative examples in humans include CD34 transcript variant 1 (nucleic acid sequence: NM_001025109.2, amino acid sequence: NP_001020280.1) and CD34 transcript variant 2 (nucleic acid sequence: NM_001773.3, amino acid sequence: NP_001764.1).

As used herein, the term "tumor necrosis factor alpha" or an abbreviation thereof, "TNF-α", refers to a protein that is mainly secreted by a macrophage and is a multifunctional proinflammatory cytokine belonging to the tumor necrosis factor (TNF) superfamily which is also known as TNF, DIF, TNFA, TNFSF2, and TNLG1F. TNF-α can function via receptors TNFRSF1A/TNFR1 and TNFRSF1B/TNFBR and is involved in regulating a wide range of biological processes such as cell growth, differentiation, apoptosis, lipid metabolism, and blood coagulation. Representative examples in humans include nucleic acid: NM_000594.4, amino acid sequence: NP_000585.2.

As used herein, the term "proinsulin" refers to a precursor protein of insulin. Proinsulin is processed in an endoplasmic reticulum of a pancreatic β-cell, and the C-peptide region is removed in an immature secretory granule, such insulin composed of an A chain and a B chain is produced. Representative examples in humans include proinsulin transcript variant 1 (nucleic acid sequence: NM_000207.3, amino acid sequence: NP_000198.1), proinsulin transcript variant 2 (nucleic acid sequence: NM_001185097.2, amino acid sequence: NP_001172026.1), proinsulin transcript variant 3 (nucleic acid sequence: NM_001185098.1, amino acid sequence: NP_001172027.1), and proinsulin transcript variant 4 (nucleic acid sequence: NM_001291897.2, amino acid sequence: NP_001278826.1).

As used herein, the term "c-Kit" refers to a type 3 transmembrane receptor for MGF (a mast cell growth factor, also known as a stem cell factor), which is also known as PBT, SCFR, KIT, CD117, or MASTC. Representative examples in humans include Kit isoform 1 precursor (nucleic acid sequence: NM_000222.2; amino acid sequence: NP_000213.1) and Kit isoform 2 precursor (nucleic acid sequence: NM_001093772.1; amino acid sequence: NP_001087241.1).

As used herein, the term "CD20" refers to a B lymphocyte surface molecule that is a member of the transmembrane 4A gene family, which is also known as MS4A1, B 1, S7, Bp35, CD20, CVID5, MS4A2, or LEU-16, and that plays a role in the development and differentiation of B cells into plasma cells. Representative examples in humans include CD20 transcript variant 1 (nucleic acid sequence: NM_152866.2, amino acid sequence: NP_690605.1) and CD20 transcript variant 3 (nucleic acid sequence: NM_021950.3, amino acid sequence: NP_068769.2).

Unless otherwise indicated, it is understood that reference to each protein in the present specification is intended not only directed to a protein having an amino acid sequence set forth in a particular accession number (or a nucleic acid encoding the same), but also to a functional equivalent thereof.

As used herein, it is understood that the "functional equivalent" of a certain molecule encompasses a mutant or variant (for example, an amino acid sequence variant or the like) of the molecule which has a function as a feature (for example, a marker) described as used herein, those that exert the same function (not necessarily to the same degree) as the biological function of the molecule, and those that can change, upon action, into the molecule itself.

As used herein, the "functional variant" of a certain molecule encompasses a substance resulting from altering the molecule while maintaining the function of the molecule (the degree of the function may be changed). Examples of a functional variant of a binding molecule such as an antibody include a substance conjugated with other portions (labels, other functional molecules (such as proteins), or the like) and a substance fragmented so as to maintain the function of binding with a target molecule. As such, as used as used herein, a functional variant maintains the function that is the basis prior to an alteration.

As the functional equivalent of the present disclosure, an amino acid sequence in which one or more amino acids are inserted, substituted, or deleted, or added to one or both terminals thereof can be used. As used herein, the phrase "an amino acid sequence in which one or more amino acids are inserted, substituted, or deleted, or added to one or both terminals thereof' refers to an alteration with a substitution of a plurality of amino acids or the like to the extent that can occur naturally by a well-known technical method such as sitedirected mutagenesis or natural mutation.

As used herein, when referring to a certain gene or a nucleic acid molecule or a polypeptide related thereto, the term "biological function" refers to a specific function that the gene, the nucleic acid molecule, or the polypeptide can have in a living body. Examples of such a function include, but are not limited to, production of a specific antibody, enzyme activity, impartation of resistance. For such a biological activity, literatures and the like cited in the accession numbers, Entrez numbers, and the like mentioned in the table described above can be referred to, and such literatures and the like are also incorporated herein by reference. As used herein, the biological function can be exerted by "biological activity". As used herein, the term "biological activity" refers to an activity that a certain agent (for example, a polynucleotide, a protein, or the like) can have in a living body. Biological activity encompasses an activity of exerting a variety of functions (for example, transcription promoting activity), and also encompasses, for example, an activity of activating or inactivating another molecule by an interaction with a certain molecule. When two factors interact, the biological activity thereof is a bond between the two molecules and a biological change induced thereby, for example, two molecules are considered to be bound together when precipitating one molecule using an antibody results in co-precipitation of the other molecule. Therefore, observation such co-precipitation is one example of a determination approach. For example, when a certain factor is an enzyme, the biological activity thereof encompasses enzyme activity thereof. In another example, in a case where a certain factor is a ligand, binding to a receptor corresponding to the ligand is encompassed. Such biological activity can be measured by a technique that is well known in the art.

A "derivative", "analog", or "mutant" of a protein or nucleic acid used in the present specification includes, but is not intended to be limited to, molecules having a region substantially homologous to the protein or nucleic acid. Such a molecule, in various embodiments, is at least 30%, 40%, 50%, 60%, 70%, 80%, 90%, 95%, 98% or 99% identical throughout the amino acid sequence or nucleic acid sequence of the same size or in comparison to a sequence aligned by a homology computer program known in the art. Alternatively, a "derivative", "analog", or "mutant" of a nucleic acid can hybridize to the original nucleic acid under a stringent condition, moderately stringent condition, or nonstringent condition. Typically, a "derivative", "analog", or "mutant" of a protein refers to a product obtained by subjecting a naturally-occurring protein to modification such as an amino acid substitution, deletion, and addition, which is a protein exhibiting the biological function of the naturally-occurring protein, although not necessarily to the same degree. For example, the biological function of such a protein can be investigated by a suitable and available in vitro assay described in the present specification or known in the art. Although the present disclosure mainly discusses humans, it is understood that it also applies to those of other species, such as other species within primates or species of animals of other genera, and that these mammals are also within the scope of the present disclosure.

The term "activity" used in the present specification refers to a function of a molecule in the broadest sense. Activity, although not intended to be limiting, generally includes a biological function, biochemical function, physical function, and chemical function of a molecule. Examples of the activity include enzymatic activity, an ability to interact with another molecule, an ability to activate, promote, stabilize, inhibit, suppress, or destabilize a function of another molecule, stability, and an ability to localize at a specific position in a cell. When applicable, the term also relates to a function of a protein complex in the broadest sense.

The "functionally active" protein, polypeptide, fragment, or derivative as used in the present specification has a structural function, regulatory function, or biochemical function of a protein such as a biological activity. As used herein, it is understood that the "functionally active" protein, polypeptide, fragment, or derivative only needs to have at least one of the intended biological activities and the like, as long as it is for the objects of the present disclosure.

As used herein, the term "gene" refers to a factor defining a genetic trait. A gene is generally arranged in a certain order in a chromosome. A gene defining a primary structure of a protein is referred to as a structural gene, and a gene affecting the expression thereof is referred to as a regulator gene. As used herein, the term "gene" may refer to a "polynucleotide", "oligonucleotide", and "nucleic acid" (including DNA, RNA, or the like herein). The term "gene product" is a protein that is a substance which is produced based on a gene, mRNA, or the like. Thus, mRNA can also be included in the concept of a gene and can also correspond to a gene product. In addition, the term "expression of a gene" often refers to a transcription level of mRNA or the like.

As used herein, the terms "protein", "polypeptide", "oligopeptide", and "peptide" are used in the same meaning as used herein, and refer to an amino acid polymer of any length. The polymer may be linear, branched, or cyclic. An amino acid may be a naturally-occurring, non-naturally occurring, or altered amino acid. The term can also encompass those assembled into a complex of a plurality of polypeptide chains. The term also encompasses naturally-occurring or artificially altered amino acid polymers. Examples of such an alteration include disulfide bond formation, glycosylation, lipidation, acetylation, phosphorylation, and any other manipulation or alteration (for example, conjugation with a labeling component). The definition also encompasses, for example, polypeptides containing one or two or more analogs of an amino acid (for example, including non-naturally occurring amino acids and the like), peptide-like compounds (for example, peptoids), and other alterations known in the art.

As used herein, the terms "polynucleotide", "oligonucleotide", and "nucleic acid" are used in the present specification to have the same meaning, and refer to a polymer of nucleotides of any length. The terms also encompass an oligonucleotide derivative or a polynucleotide derivative. As used herein, the "nucleic acid" is also interchangeably used with a gene, cDNA, mRNA, oligonucleotide, and polynucleotide. As used herein, the "nucleotide" may be natural or non-natural.

As used herein, the term "homology" of genes refers to a degree of identity of two or more genetic sequences with respect to one another, and having "homology" generally refers to having a high degree of identity or similarity. Therefore, the identity or similarity of sequences is higher as homology of two genes is high. Whether two types of genes have homology can be investigated by a direct comparison of sequences or by a hybridization method under stringent conditions for nucleic acids. When two genetic sequences are directly compared, the genes have homology when DNA sequences are typically at least 50% identical, preferably at least 70% identical, and more preferably at least 80%, 90%, 95%, 96%, 97%, 98%, or 99% identical between the genetic sequences. Thus, as used as used herein, the term "homolog" or "homologous gene product" refers to a protein in another species, preferably mammal, exerting the same biological function as a protein constituent of a complex which will be further described in the present specification. Such a homolog may also be referred to as an "ortholog gene product".

Amino acids may be mentioned in the present specification by either their commonly known three letter symbols or their one character symbols recommended by the IUPAC-IUB Biochemical Nomenclature Commission. Similarly, nucleotides may be mentioned by their commonly recognized one character codes. As used herein, a comparison of similarity, identity, and homology of an amino acid sequence and a base sequence is calculated by using a sequence analysis tool BLAST and default parameters. For example, identity can be searched using BLAST 2.2.9 (published on May 12, 2004) of the NCBI. As used herein, a value for identity generally refers to a value obtained when aligned under the default conditions using BLAST described above. However, when a higher value is obtained by changing a parameter, the highest value is set as the value of identity. In a case where identity is evaluated in a plurality of regions, the highest value in the plurality of regions is set as the value of identity. Similarity is a numerical value calculated by taking into consideration a similar amino acid in addition to identity.

As used herein, the term "purified" substance or biological factor (for example, a nucleic acid, a protein, or the like) refers to a substance or a biological factor in which at least a part of a factor naturally accompanying the biological factor is removed. Thus, the purity of the biological factor in the purified biological factor is generally higher than the purity in the normal state of the biological factor (that is, concentrated). The term "purified" as used in the present specification refers to the presence of preferably at least 75% by weight, more preferably at least 85% by weight, still more preferably at least 95% by weight, and most preferably at least 98% by weight of a biological factor of the same type. The substance used in the present disclosure is preferably a "purified" substance.

As used herein, the term "corresponding" amino acid or nucleic acid refers to an amino acid or a nucleotide which has or is expected to have, in a certain polypeptide molecule or polynucleotide molecule, a similar action as a predetermined amino acid or nucleotide in a reference polypeptide or a polynucleotide for comparison, and, particularly in the case of enzyme molecules, refers to an amino acid which is present at a similar position in an active site and makes a similar contribution to catalytic activity. For example, for an antisense molecule, it can be a similar moiety in an ortholog corresponding to a specific moiety of the antisense molecule. A corresponding amino acid can be a specific amino acid subjected to, for example, cysteination, glutathionylation, S-S bond formation, oxidation (for example, oxidation of a methionine side chain), formylation, acetylation, phosphorylation, glycosylation, myristylation, or the like. Alternatively, a corresponding amino acid can be an amino acid responsible for dimerization. Such a "corresponding" amino acid or nucleic acid may be a region or a domain over a certain range. Accordingly, it is referred in the present specification as a "corresponding" region or domain in such a case.

As used herein, the term "corresponding" gene (for example, a polynucleotide sequence or molecule) refers to a gene (for example, a polynucleotide sequence or molecule) in a certain species which has or is expected to have a similar action as a predetermined gene in a reference species for comparison. When a plurality of genes having such an action are present, the corresponding gene refers to a gene having the same evolutionary origin. Accordingly, a gene corresponding to a certain gene may be an ortholog of such a gene. Such a corresponding gene can be identified by using a technique that is well known in the art. Therefore, for example, a corresponding gene in a certain animal (for example, a mouse or rat) can be found by search on a sequence database for the animal using the sequence of a reference gene of the corresponding gene (for example, gene of humans) as a query sequence.

As used herein, the term "fragment" refers to a polypeptide or polynucleotide having a sequence length of 1 to n-1 with respect to the full length polypeptide or polynucleotide (having a length n). The length of a fragment can be appropriately changed in accordance with the object. Examples of a lower limit of such a length include 3, 4, 5, 6, 7, 8, 9, 10, 15, 20, 25, 30, 40, 50, and more amino acids for a polypeptide. Lengths represented by an integer that is not specifically listed herein (for example, 11 and the like) also can be suitable as the lower limit. In addition, examples of the length include 5, 6, 7, 8, 9, 10, 15, 20, 25, 30, 40, 50, 75, 100, and more nucleotides for a polynucleotide. Lengths represented by an integer that is not specifically listed herein (for example, 11 and the like) also can be suitable as the lower limit. As used herein, it is understood that such a fragment is within the scope of the present disclosure, for example, when a full length version functions as a marker, as long as the fragment itself also functions as a marker. In the present disclosure, a fragment of a molecule is a substance (typically, a polypeptide) having any region of the molecule, which does not need to have the biological function of a naturally-occurring molecule as long as the fragment can be used for the object of the present disclosure (for example, therapy, detection, diagnosis, or the like).

In the present disclosure, the term "expression" of a gene, a polynucleotide, a polypeptide, or the like refers to the gene or the like being subjected to a certain action in vivo to be converted into another form. Preferably, the expression refers to a gene, a polynucleotide, or the like being transcribed and translated into a form of a polypeptide. However, the expression may also be an aspect of expression that is transcribed to produce mRNA. More preferably, such a polypeptide form can be a form which has undergone posttranslation processing (derivative as referred to in the present specification). For example, the expression level of the molecule can be determined by any method. Specifically, the expression level can be known by evaluating the amount of mRNA, the amount of protein, or the biological activity of protein of the molecule.

As used herein, the term "expression level" refers to the amount of polypeptide, mRNA, or the like expressed in a target cell, tissue, or the like. Examples of such an expression level include an expression level of the polypeptide of the present disclosure at the protein level of the polypeptide of the present disclosure evaluated by any appropriate method including an immunological measurement method such as an ELISA method, an RIA method, a fluorescent antibody method, a Western blotting method, or an immunohistochemical staining method using the antibody of the present disclosure, and an expression level at an mRNA level of the polypeptide used in the present disclosure evaluated by any appropriate method including a molecular biological measurement method such as a Northern blotting method, a dot blotting method, or a PCR method. The term "change in expression level" refers to an increase or decrease in expression level of the polypeptide used in the present disclosure at a protein level or mRNA level evaluated by any appropriate method including the immunological measurement method or molecular biological measurement method described above. A variety of detection or diagnosis based on a marker can be performed by measuring an expression level of a certain marker.

As used herein, the term "marker (substance, protein, or gene (nucleic acid))" refers to a substance that can be an indicator for tracking whether a subject is in or at risk of being in a certain state (for example, the cell type, normal cell state, disease state, disorder state, or proliferative capacity, the level of differentiation state, or the like). Examples of such a marker include a blood glucose level, a gene (nucleic acid = DNA level), a gene product (such as mRNA, protein, or the like), a metabolite, and an enzyme. In the present disclosure, detection, diagnosis, preliminary detection, prediction, or prediagnosis of a certain state (such as diabetes mellitus) can be achieved using a drug, agent, factor, or means specific to a marker associated with the state, or a composition, kit, system, or the like containing the same.

As used herein, the term "means" to any tool that can achieve a certain purpose (for example, detection, diagnosis, treatment, or prevention), and in particular, as used herein, the term "means for selectively recognizing (detecting)" refers to means capable of recognizing (detecting) a certain subject differently from others.

As used herein, "detection" or "quantification" of polynucleotide or polypeptide expression can be accomplished using a suitable method including, for example, measurement of mRNA and an immunological measurement method, including a bond or interaction to a marker detection agent. Examples of a molecular biological measurement method include a Northern blotting method, a dot blotting method, and a PCR method. Examples of the immunological measurement method include an ELISA method using a microtiter plate, an RIA method, a fluorescent antibody method, a luminescence immunoassay (LIA), an immunoprecipitation method (IP), an immunodiffusion method (SRID), a turbidimetric immunoassay (TIA), a Western blotting method, and an immunohistochemical staining method. In addition, examples of a quantification method include an ELISA method and an RIA method. It can also be performed by a genetic analysis method using an array (for example, a DNA array or a protein array). DNA arrays are outlined extensively in ("DNA microarray and latest PCR method", Cell Engineering Edition, edited by Shujunsha Co., Ltd.). Protein arrays are described in detail in Nat Genet. 2002 Dec; 32 Suppl: 526-32. Examples of a method for analyzing gene expression include, but are not limited to, RT-PCR, a RACE method, an SSCP method, an immunoprecipitation method, a two-hybrid system, and in vitro translation, in addition to the methods described above. Such additional analysis methods are described, for example, in Genome Analysis Experimental Method, Yusuke Nakamura Lab Manual, Edited by Yusuke Nakamura Yodosha (2002), and the like, and the entirety of the descriptions is incorporated herein by reference.

As used herein, the term "probe" refers to a substance serving as a means of search used in a biological experiment such as screening in vitro and/or in vivo, and examples thereof include, but are not limited to, a nucleic acid molecule containing a specific base sequence or a peptide containing a specific amino acid sequence, and a specific antibody or a fragment thereof. As used herein, the probe is used as a marker detection means.

As used herein, the term "label" refers to the presence (for example, a substance, energy, an electromagnetic wave, and the like) for identifying a molecule or substance to be targeted from others. Examples of such a labeling method include a radioisotope (RI) method, a fluorescence method, a biotin method, and a chemiluminescence method. When a plurality of markers or factors of the present disclosure or means for capturing the markers are labeled by a fluorescence method, the markers are labeled with fluorescent substances having different maximum fluorescence emission wavelengths.

As used as used herein, the term "tag" refers to a substance for sorting a molecule by a specific recognition mechanism such as a receptor-ligand, and more specifically, a substance that serves as a binding partner for binding a specific substance (for example, having a relationship such as biotin-avidin or biotin-streptavidin), and may be included in the category of a "label". Therefore, for example, a specific substance to which a tag is bound can be sorted by bringing a substrate to which a binding partner of the tag sequence is bound into contact. Such a tag or label is well known in the art. Representative tag sequences include, but are not limited to, a myc tag, a His tag, HA, an Avi tag, and the like. Such a tag may be bound to the marker or marker detection agent of the present disclosure.

As used herein, a "drug", "agent", or "factor" (all the terms correspond to an "agent" in English) is used interchangeably in abroad sense, and may be any substance or other elements (for example, energy such as light, radioactivity, heat, and electricity) as long as the intended object can be achieved. Examples of such a substance include, but are not limited to, a cell (for example, a T cell), a protein, a polypeptide, an oligopeptide, a peptide, a polynucleotide, an oligonucleotide, a nucleotide, a nucleic acid (including, for example, DNA such as cDNA or genomic DNA, RNA such as mRNA), a polysaccharide, an oligosaccharide, a lipid, an organic small molecule (for example, a hormone, a ligand, an information transmitting substance, an organic small molecule, a molecule synthesized by combinatorial chemistry, a small molecule that can be used as a medicine (for example, a small molecule ligand or the like), or the like) and a composite molecule thereof.

As used herein, the term "kit" refers to a unit generally providing portions to be provided (for example, an inspection drug, a diagnostic drug, a therapeutic drug, a manual, and the like) that are usually divided into two or more sections. The form of the kit is preferred when it is intended to provide a composition that should not be provided in a mixed state for stability or the like, but is preferably mixed immediately before use. Such a kit preferably includes an instruction or manual describing how to use the provided portions (for example, an inspection drug, a diagnostic drug, a therapeutic drug, or the like) or how a reagent should be processed. When the kit is used in the present specification as a reagent kit, the kit generally includes an instruction or the like describing how to use an inspection drug, a diagnostic drug, a therapeutic drug, and the like.

As used herein, the "instruction" is a document with an explanation of the method of use of the present disclosure for a physician or other users. The instruction has a description of the detection method of the present disclosure, the method of use of the diagnostic drug, administration of the medicine, or the like. In addition, the instruction may have a description instructing oral administration or esophageal administration (for example, by injection or the like) as an administration site. The instruction is prepared in accordance with a format defined by the regulatory agency of the country in which the present disclosure is practiced (for example, the Ministry of Health, Labor and Welfare in Japan, Food and Drug Administration (FDA) in the United States or the like), with an explicit description showing approval by the regulatory agency. The instruction is a so-called package insert and is usually provided in, but is not limited to, a paper medium. For example, the instruction may also be provided in a form such as an electronic medium (for example, a web site or an e-mail provided on the Internet).

As used as used herein, the term "amount" of an analyte or the like in a sample generally refers to an absolute value that reflects the mass of the analyte that may be detected in the volume of the sample. However, the amount is also intended as a relative amount as compared to the amount of another analyte. For example, the amount of the analyte in the sample can be an amount that is greater than a control value or a normal value of the analyte that is generally present in the sample.

As used as used herein, the term "level" of the analyte or the like in the sample generally refers to an absolute value that reflects the value of activity or the like of the analyte when the analyte is a subject exerting a function such as an enzyme. However, the level is also intended as a relative level as compared to the level of another analyte. For example, the level of the analyte in the sample can be a level that is greater than a control value or a normal value of the analyte that is generally present in the sample.

The term "about" as used in the present specification refers to the indicated value plus or minus 10%. Note that even when "about" is not explicitly indicated, it can be interpreted synonymously with a value with "about".

### (Outline of present disclosure)

The present inventors have found a novel aspect in which excellent diabetes mellitus treatment effects can be obtained by combining HDAC regulation (for example, HDAC inhibition or activation) and blood glucose control. The present disclosure provides a novel treatment strategy for diabetes mellitus and/or an associated disease thereof based on the novel treatment strategy for diabetes mellitus.

### (Preferred embodiments)

Preferred embodiments of the present disclosure will be described below. It is understood that the embodiments provided below are provided for a better understanding of the present disclosure, and that the scope of the present disclosure should not be limited to the following description. Therefore, it is apparent that those skilled in the art can appropriately make modifications within the scope of the present disclosure in view of the description in the present specification. It is also understood that the following embodiments of the present disclosure may be used alone or in combination.

(Treatment and/or prevention of diabetes mellitus and/or disease, disorder, and/or symptom associated with diabetes mellitus by combination of HDAC regulation and control of blood glucose)

In one aspect, the present disclosure provides a treatment and/or prevention of diabetes mellitus and/or a disease, disorder, and/or symptom associated with diabetes mellitus by combining regulation of histone deacetylase (HDAC) (for example, inhibition or activation of HDAC) and control of blood glucose. The treatment and/or prevention may be achieved by any means, such as implementation of a method, provision of a composition, combined product, kit, or system for the purpose. In one embodiment, administering an HDAC regulator to a subject in a state in which blood glucose is controlled treats and/or prevents diabetes mellitus and/or a disease, disorder, and/or symptom associated with diabetes mellitus. In one embodiment, administering an HDAC regulator to a subject in combination with a blood glucose control treatment treats and/or prevents diabetes mellitus and/or a disease, disorder, and/or symptom associated with diabetes mellitus. In the present disclosure, regulation of HDAC can be achieved by appropriately activating, inhibiting, or otherwise regulating individual HDACs based on the description in the present disclosure.

In one embodiment, the state in which blood glucose is controlled can be determined by fasting blood glucose, a blood glucose level at 2 hours of an oral glucose tolerance test, and/or a casual blood glucose level. In one embodiment, a state in which fasting blood glucose is less than 150 mg/dL, less than 145 mg/dL, less than 140 mg/dL, less than 135 mg/dL, less than 130 mg/dL, less than 126 mg/dL, less than 120 mg/dL, less than 115 mg/dL, or less than 110 mg/dL can be a state in which blood glucose is controlled. In one embodiment, a state in which a blood glucose level at 2 hours of an oral glucose tolerance test (for example, 75 g OGTT) is less than 250 mg/dL, less than 240 mg/dL, less than 230 mg/dL, less than 220 mg/dL, less than 210 mg/dL, less than 200 mg/dL, less than 190 mg/dL, less than 180 mg/dL, less than 170 mg/dL, less than 160 mg/dL, or less than 150 mg/dL can be a state in which blood glucose is controlled. In one embodiment, a state in which a casual blood glucose level (a blood glucose level measured regardless of meal time, for example, a blood glucose level measured within 1 hour after a meal) is less than 250 mg/dL, less than 240 mg/dL, less than 230 mg/dL, less than 220 mg/dL, less than 210 mg/dL, less than 200 mg/dL, less than 190 mg/dL, less than 180 mg/dL, less than 170 mg/dL, less than 160 mg/dL, or less than 150 mg/dL can be a state in which blood glucose is controlled. In one embodiment, the state in which blood glucose is controlled may be determined by one measurement result, may be determined by a plurality of measurement results, or may be determined by measurement results for a certain period (for example, 1 day, 2 days, 3 days, 4 days, 5 days, 6 days, 7 days, 2 weeks, 3 weeks, 4 weeks, or the like).

In one embodiment, a blood glucose level in a subject may be measured in the treatment and/or prevention of diabetes mellitus and/or a disease, disorder, and/or symptom associated with diabetes mellitus by combining regulation of HDAC and control of blood glucose. For example, the measurement of the blood glucose level may be performed before, after, and/or during the treatment period of regulating HDACs and/or controlling blood glucose. In one embodiment, outside of the treatment period, the measurement of the blood glucose level may be performed once every 1 day, 2 days, 3 days, 4 days, 5 days, 6 days, 7 days, 2 weeks, 3 weeks, or 4 weeks. In one embodiment, within the treatment period, the measurement of the blood glucose level may be performed once every 1 day, 2 days, 3 days, 4 days, 5 days, 6 days, or 7 days, or 1 to 3 times per day. In a specific embodiment, outside the treatment period, the measurement of the blood glucose level is performed once a week, and within the treatment period, the measurement of the blood glucose level is performed at the same frequency as the treatment (for example, when the HDAC regulator is administered once every 3 days, once every 3 days).

In one embodiment, HDAC regulation in the treatment and/or prevention of the present disclosure may be performed using an HDAC regulator. The HDAC to be regulated may be any of class I, class IIa, class IIb, class III, class IV, HDAC1 to 11 and SIRT1 to 7. In one embodiment, HDAC regulation inhibits class I, class II, and/or class IV HDAC. In one embodiment, HDAC regulation inhibits one or more of HDAC1, HDAC2, HDAC3, HDAC4, HDAC5, HDAC6, HDAC7, HDAC8, HDAC9, and HDAC 10. In one embodiment, HDAC regulation activates one or more of SIRT1, SIRT2, SIRT3, SIRT4, SIRT5, SIRT6, and SIRT7. In one embodiment, any combination of these inhibition and activation is also intended and is also within the scope of the present disclosure. In one embodiment, HDAC regulation activates SIRT1 and/or SIRT6. The HDAC of the regulatory target may be selectively regulated or may be regulated together with other HDACs. It can be preferable for SIRT to have increased activity in organs other than bone marrow, and in the present disclosure it can be suitable for class III HDAC not to be inhibited and/or activated. For example, activation of SIRT1 and SIRT6 may reduce oxidative stress by suppressing inflammation in diabetes mellitus. A drug which does not inhibit class III HDAC like givinostat and broadly inhibit class I, II and IV HDACs can be particularly preferred. Without wishing to be bound by a particular theory, each HDAC is expected to have functions as shown in the table below in diabetes mellitus, and therefore, regulation of HDAC may be selected to control these functions.

**[Table 2]**

| Gene | Change observed in abnormal hematopoietic stem cells | Expected effects of increase on diabetes mellitus |
|---|---|---|
| Hdac1 | No change | Decreased sugar uptake |
| Hdac2 | Decrease | Decreased sugar uptake |
| Hdac3 | Increase | Increased sugar release |
| Hdac4 | Increase | Decreased sugar uptake/increased sugar release |
| Hdac5 | No change | Decreased sugar uptake/increased sugar release |
| Hdac6 | No change | Deterioration of glucose tolerance |
| Hdac7 | No change | Decreased insulin secretion |
| Hdac8 | Increase | Deterioration of glucose tolerance |
| Hdac9 | Decrease | Deterioration of glucose tolerance |
| Hdac10 | No change | No report |
| Hdac11 | Decrease | Deterioration of glucose tolerance |
| Sirt1 | Increase | Prevention of complications |
| Sirt2 | No change | Decrease in oxidative stress |
| Sirt3 | No change | Increased mitochondrial function |
| Sirt4 | No change | Increased mitochondrial function |
| Sirt5 | No change | Increased mitochondrial function |
| Sirt6 | Increase | Increased DNA repair |
| Sirt7 | No change | Decrease in ER stress |

For no change, increase, or decrease in the above table, one or more may be different, and the pattern may exhibit exactly the same variation as described in the table, or one or more may vary in a different manner. As a different mode in abnormal hematopoietic stem cells, it is considered that the increase does not change or decreases, the increase does not change or decreases, or the decrease does not change or increases, and embodiments based thereon are also intended to be within the scope of the present disclosure.

The HDAC regulator can be any substance such as a small molecule compound, an inhibitory nucleic acid (including those intended for gene therapy), or an antibody. Examples of the HDAC regulator include, but are not limited to, fingolimod (FTY720), givinostat, vorinostat (suberoylanilide hydroxamic acid, SAHA), belinostat, panobinostat, resminostat, abexinostat, mocetinostat, trichostatin A (TSA), romidepsin, apicidin, chidamide, entinostat, tacedinaline, tucidinostat, pracinostat (SB939), quisinostat, tefinostat, rocilinostat, fimepinostat, nanatinostat, domatinostat, trapoxin (TPX), cyclic hydroxamic acid-containing peptide 1 (CHAP1), valproate (VPA), sodium butyrate (NaBu), 4-phenylbutyric acid, OBP-801, ME-344, CG200745, MPT0E014, RGFP966 (MCE, HY-13909), SK-7041, scriptaid, MC1568, MHY2256, ricolinostat (ACY-1215), Tubacin (S2239), Tubathian A, Tubastatin A, Pojamide, AN7, CUDC-101, KD5170, R36465, SOU-HDAC42, LBH589, SFN, phenyl-hexyl isothiocyanate, dacinostat (LAQ824), m-carboxycinnamic acid bis-hydroxamide (CBHA), azelaoyl-bis-hydroxamic acid (ABHA) (N,N'-dihydroxynonanediamide), AR-42, suberoyl bis-hydroxamic acid (SBHA), (pivaloyloxy)methyl butyrate (pivanex, AN-9), diallyl trisulfide (DATS), PCI-24781, nicotinamide, tenovin-1, tenovin-6, sirtinol, EX-527, HBI-8000, kevetrin, CHR-3996, 4SC202, WW437, α-ketoamides, heterocyclic ketones, cambinol, I-7ab, C149, depudecin, SEN 196, COMPOUND6J, JGB1741, JQ1, I-BET151, BY27, CUDC907, DWP0016, MC1568, W2, RGFP109, tinostamustine (EDO-S101), J22352, M344, ITF-3056, TMP195 (TFMO 2), MI192, Santacruzamate A(CAY10683), ACY-738, BML-281 (CAY10603), LTB2, CKD-506, WK2-16, PCI-34051, ESM-HDAC528, Merck60, (N-hydroxy-7-2-naphthylthio)heptanomide (HNHA), KBH-A42, AGK2, sulforaphane, MS-275, resveratrol, APHA, curcumin, remetinostat, citarinostat, dacinostat, droxinostat, BRD3308, nimbolide, betaine, and chrysophanol. In a preferred embodiment, the HDAC regulator is selected from fingolimod (FTY720), givinostat, trichostatin A, vorinostat, belinostat, panobinostat, resminostat, abexinostat, quisinostat, pracinostat, rocilinostat, nanatinostat, dacinostat, (N-hydroxy-7-2-naphthylthio)heptanomide, valproate, CG200745, scriptaid, KBH-A42, romidepsin, sodium butyrate, 4-phenylbutyric acid, SK-7041, WW437, entinostat, tacedinaline, mocetinostat, (pivaloyloxy)methyl butyrate, trapoxin, α-ketoamides, heterocyclic ketones, diallyl trisulfide, ricolinostat, Tubacin, azelaoyl-bis-hydroxamic acid, and m-carboxycinnamic acid bis-hydroxamide. In particular, an HDAC regulator capable of reaching the bone marrow, such as givinostat, can also act on abnormal stem cells that are present in the bone marrow and cause diabetes mellitus, and thus may be preferably used in the present disclosure.

It is considered that the abnormal stem cells adhere to and exist in a niche in the bone marrow, are released from the niche only when cell proliferation is performed, and adhere to and hide from the niche again when proliferation is terminated. Therefore, in order to efficiently act the HDAC regulator, a hematopoietic stem cell migration agent, for example, a CXCR4 inhibitor (for example, plerixafor (AMD3100)), an epidermal growth factor receptor (EGFR) inhibitor (for example, gefitinib, erlotinib, afatinib, osimertinib, or the like), a granulocyte colony-stimulating factor (G-CSF) stimulator (for example, filgrastim, nartograstim, lenograstim, pegfilgrastim, or the like), a CXCR2 stimulator (for example, GROβ (MIP2)), and the like may be further used to release abnormal stem cells present in the bone marrow from the niche.

The control of blood glucose in the treatment and/or prevention of the present disclosure can refer to both a state in which control of blood glucose is achieved and a treatment for controlling blood glucose, and examples of the treatment for controlling blood glucose include administration of a hypoglycemic agent, dietary restriction, and nutrient infusion control. In one embodiment, the hypoglycemic agent is selected from the group consisting of insulin, an insulin analog, a biguanide, an insulin secretagogue (a sulfonylurea drug (SU drug) or the like), a fast-acting insulin secretagogue (a glinide drug or the like), an incretin-related drug (a DPP-4 inhibitor, a GLP-1 receptor agonist, or the like), a sugar absorption regulator (α-glucosidase inhibitor (α-GI)), an insulin resistance-ameliorating agent (a thiazolidine drug), a urine glucose evacuant (a SGLT2 inhibitor), and a combination of two or more thereof. In one embodiment, the hypoglycemic agent can be selected from the group consisting of insulin, degludec, glargine, lispro, detemir, aspart, metformin, phenformin, buformin, tolbutamide, glibenclamide, gliclazide, chlorpropamide, tolazamide, acetohexamide, glyclopyramide, glimepiride, glybuzole, glucotrol, saroglitazar, aleglitazar, muraglitazar, tesaglitazar, ragaglitazar, metaglidasen, naveglitazar, reglitazar, farglitazar, peliglitazar, sodelglitazar, indeglitazar, nateglinide, repaglinide, mitiglinide, gliptin, sitagliptin, vildagliptin, saxagliptin, linagliptin, gemigliptin, anagliptin, teneligliptin, alogliptin, trelagliptin, dutogliptin, omarigliptin, berberine, lupeol, liraglutide, exenatide, lixisenatide, albiglutide, voglibose, acarbose, miglitol, emiglitate, pioglitazone, rosiglitazone, edaglitazone, balaglitazone, lobeglitazone, ciglitazone, darglitazone, englitazone, netoglitazone, rivoglitazone, troglitazone, leriglitazone, empagliflozin, canagliflozin, dapagliflozin, ipragliflozin, tofogliflozin, sergliflozin etabonate, remogliflozin etabonate, ertugliflozin, dulaglutide, teduglutide, taspoglutide, luseogliflozin, semaglutide, a biphasic protamine crystalline insulin analog aqueous suspension injection, an amorphous insulin zinc aqueous suspension injection, lenteinsulin (insulin zinc aqueous suspension injection), an isophene insulin aqueous suspension injection, a biosynthetic human biphasic isophene insulin aqueous suspension injection, ultralente insulin (crystalline insulin zinc aqueous suspension injection), GLP-1, a GLP-1 analog, GIP, and a combination of two or more thereof.

In one embodiment, the treatment of controlling blood glucose can be performed to achieve a state in which blood glucose is controlled as described in the present specification. The dietary restriction as a treatment for controlling blood glucose is usually understood by those skilled in the art, but for example, reducing the total calories ingested, reducing the carbohydrates ingested, adopting carbohydrates that are difficult to be ingested, and the like can be implemented in combination with adjustment of meal timing as necessary. The nutrient infusion control as a treatment for controlling blood glucose is usually understood by those skilled in the art, but may be, for example, lowering carbohydrates in infusion.

The HDAC regulation as described in the present specification can remove abnormal hematopoietic stem cells, but immediately after removal of abnormal hematopoietic stem cells, pancreatic β-cells (or thymus, which further prevents β-cell injury) cannot yet recover, thus resulting in insufficient blood glucose control and hyperglycemia. As described as used herein, since hyperglycemia can promote the reappearance of abnormal hematopoietic stem cells, it can be advantageous to suppress hyperglycemia until pancreatic β-cells (or thymus, which further prevents β-cell injury) recover. The regulation of HDAC and the control of blood glucose may be performed over the same period of time, or may be performed so that the regulation of HDAC and the control of blood glucose partially overlap, or the blood glucose may be controlled after the regulation of HDAC, or HDAC may be regulated after the control of blood glucose. The control of blood glucose may be sustained until the pancreatic β-cells (or thymus, which further prevents β-cell injury) recover to such an extent that self-control of blood glucose is possible. In a case where the regulation of HDAC and the control of blood glucose are controlled by drugs, the drugs may be administered separately or together, may be provided in one composition, or may be provided individually in another composition.

In one embodiment, the treatment and/or prevention of the present disclosure is performed on a subject diagnosed as having abnormal hematopoietic stem cells (HSCs). In one embodiment, during a treatment period of the treatment and/or prevention of the present disclosure and/or after the treatment period, a subject is diagnosed as having abnormal hematopoietic stem cells (HSCs). The characteristics of abnormal hematopoietic stem cells (HSC) are described in detail elsewhere in the present specification.

In one embodiment, the treatment and/or prevention of the present disclosure may be a treatment and/or prevention of diabetes mellitus. In one embodiment, the treatment and/or prevention of the present disclosure may be a treatment and/or prevention of complications of diabetes mellitus, for example, a neurological disorder, nephropathy, a hepatic disorder, retinopathy, fatty liver, a gastrointestinal disorder, delayed fracture healing, an eating disorder, and a skin disorder.

The treatment and/or prevention of the present disclosure may be combined with any known treatment and/or prevention treatment or means (for example, a treatment and/or prevention treatment or means of diabetes mellitus and/or a disease, disorder, and/or symptom associated with diabetes mellitus).

In one embodiment, the present disclosure provides a cure for diabetes mellitus and/or a disease, disorder, and/or symptom associated with diabetes mellitus. The treatment method described in the present specification can allow not only a delay of diabetes mellitus, but also a cure of diabetes mellitus. In one embodiment, the cure of diabetes mellitus and/or a disease, disorder, and/or symptom associated with diabetes mellitus results in a condition in which the disease, disorder, and/or symptom is not diagnosed for at least 1 month, 3 months, 6 months, 1 year, 2 years, or 5 years after the end of treatment. The cure of diabetes mellitus and/or a disease, disorder, and/or symptom associated with diabetes mellitus may be determined based on a normalization in a response of a blood glucose level by a glucose tolerance test, which provides diagnostic criteria for diabetes mellitus, as well as an ability to secrete insulin with an auxiliary diagnostic value, and a C-peptide secretion response by a glucagon tolerance test.

### (Mechanism of treatment and/or prevention)

FIG. 1 schematically illustrates the types of bone marrow cells identified by the inventors as those involved in the onset of diabetes mellitus, representative characteristics thereof, and involvement with diabetes mellitus. In general, cells that produce TNF-α are cells derived from blood, and it is unlikely that the main cells of an organ directly produce TNF-α. When investigating the origin of abnormal cells that produce TNF-α, hematopoietic stem cells (HSCs) expressed TNF-α, and TNF-α was not expressed in the most undifferentiated long term (LT)-HSC, but was expressed in short term (ST)-HSC, and expression was localized in a CD106 positive fraction of ST-HSC. ST-HSC expressing the CD106 and TNF-α also had variable expression of HDAC (HDAC 2, 3, 4, 8, 9, 11, SIRT1, 6, and the like). The regulation of HDAC can be advantageous as CD106 and TNF-α can also be expressed in normal cells with important functions and CD106 can also be expressed in normal stem cells.

Without wishing to be bound by any theory, the mechanism by which abnormal cells develop type 2 diabetes mellitus is schematically illustrated in FIG. 2. CD106/TNF-α positive abnormal hematopoietic stem cells found in STZ mice were also found in high-fat diet mice, which are a distinct type of type 2 diabetes mellitus model. In both STZ mice and high-fat diet mice, deficient insulin secretion occurs due to reduced insulin sensitivity (1), which causes hyperglycemia and promotes production of abnormal CD106/TNF-α positive stem cells in the bone marrow. It is considered that abnormal expression of a histone deacetylase group occurs in abnormal stem cells, and accordingly, various gene expression abnormalities occur. As one of them, abnormal stem cells that simultaneously express proinsulin (a precursor of insulin that does not act as a hormone) and TNF-α appear (2). These cells also migrate to the thymus where they become abnormal autoantigen-presenting cells that simultaneously present proinsulin and TNF-α (3). Therefore, expression of TNF-α in the thymus leads to atrophy of the thymus, T cells to kill abnormal cells co-expressing CD106/TNF-α/proinsulin are deleted in a negative selection (4), and CD106/TNF-α/proinsulin positive abnormal hematopoietic stem cells that have undergone immune tolerance increase in the bone marrow and migrate systemically. Once established as stem cells in the bone marrow, this abnormality does not disappear semi-permanently. As a result, the abnormal stem cells migrate from the bone marrow to the pancreatic islets to generate CD106/TNF-α/proinsulin positive vascular endothelial cells in capillaries of the pancreatic islets via cell fusion (5), and when TNF-α is expressed, β-cell progenitor cells are damaged, leading to further loss of insulin-secreting cells (6). In addition, abnormal stem cells migrate to various organs, resulting in abnormal endothelial cell function and TNF-α production in various organs (7), leading to the onset and progression of complications (8). Then, it is considered that abnormal stem cells in the bone marrow reside in a niche (refers to an environment in which stem cells can maintain their properties in vivo, and usually refers to mesenchymal cells that adhere to maintain stem cells) as a memory for maintaining memory of type 2 diabetes mellitus, and create a refractory disease state (2). These series of abnormalities can be suppressed by (A) removing abnormal stem cells in the bone marrow and (B) normalizing the blood glucose level by externally administering insulin for a certain period until the insulin secretion function recovers until the blood glucose level can be normalized in order to prevent the appearance of new abnormal stem cells, and it is considered that type 2 diabetes mellitus can be cured.

Similarly, the mechanism by which abnormal cells develop type 1 diabetes mellitus is schematically illustrated in FIG. 3. In the type 1 diabetic mouse model (NOD mouse), CD106/TNF-α positive cells that also appeared in type 2 appear. Furthermore, from progenitor cells of the B cell line, cells that produce autoantibodies that damage β-cells appear (1). Due to the autoantibodies released from these cells, β-cells gradually decrease (2). In addition, some of the abnormal B-cells migrate to the thymus, produce autoantibodies, and attack and eliminate pancreatic islet autoantigen-presenting cells such as proinsulin and GAD (3). As a result, the thymus loses the ability of negative selection of pancreatic islet attack type T cells, and allows migration of these T cells to the periphery (4). Self-attacking T cells that have migrated into the pancreatic islets injure β-cells, deplete insulin secretion at once, and type 1 diabetes mellitus develops (5). At the same time, progenitor cells of autoantigen-presenting cells expressing insulin mRNA present in the bone marrow are also attacked by the T cells and completely eliminated (6), and cells presenting proinsulin and GAD are not supplied to the thymus (7). Persistent hyperglycemia explosively increases CD106/TNF-α positive cells in the ST-HSP fraction in the bone marrow (8), and in the thymus (9) or pancreatic islets (10), proliferation of abnormal vascular endothelium derived from these cells and catastrophic tissue destruction of β-cell progenitor cells (11) are induced. In addition, it is also considered that endothelial dysfunction (12) occurs in other peripheral organs as in type 2, and various complications specific to diabetes mellitus progress due to cell fusion (13) and the like. The decisive difference between type 1 diabetes mellitus and type 2 diabetes mellitus is that cells expressing insulin or proinsulin proteins in the thymus and pancreatic islets are completely destroyed. These series of abnormalities can be suppressed by (A) removing abnormal stem cells in the bone marrow and (B) normalizing the blood glucose level by externally administering insulin for a certain period until the insulin secretion function recovers until the blood glucose level can be normalized in order to prevent the appearance of new abnormal stem cells, and it is considered that type 1 diabetes mellitus can be cured. Here, since the removal of the abnormal stem cells in (A) also results in the removal of the abnormal hematopoietic stem cells that cause the cells genetically expressing autoantibodies that have appeared in (C), it is considered that remission of type 1 diabetes mellitus can be achieved by the same treatment as in type 2 diabetes mellitus.

Without wishing to be bound by any particular theory, it is considered that a description of the diabetes mellitus treatment of the present disclosure, focusing on regulation of HDAC, will be as follows.
1) In the hyperglycemic state, the blood glucose level in the bone marrow also increases, and abnormal vascular endothelial progenitor cells expressing proinsulin and TNF-α appear. The HDAC regulation suppresses the formation of these stem cells in the bone marrow, and at the same time, apoptosis of the formed abnormal cells is also induced. On the other hand, when the blood glucose level remains high, the desired treatment effect is impaired because abnormal stem cells appear again.
2) The HDAC regulation alone has no effect on increasing the number of insulin-secreting cells.
3) The HDAC regulation alone suppresses abnormal cells by apoptosis induction or the like, and reduces inflammatory cytokines such as TNF-α released by abnormal cells. As a result, insulin resistance is lowered, and an effect of indirectly lowering a blood glucose level can also be obtained. This is also supported by the reduction in blood glucose level over time as compared to untreated STZ diabetes mellitus in a case of a treatment with only an HDAC regulator without a treatment with insulin, as illustrated in FIG. 6 and the like. However, since the number of insulin-secreting cells is not directly increased, it is considered that the blood glucose level does not decrease so much in type 1 diabetes mellitus in which insulin secretion decreases or type 2 diabetes mellitus in which insulin is no longer secreted due to progression.
4) In a case where the fasting blood glucose level of type 2 diabetes mellitus is not significantly high, due to the effects of HDAC regulation described above alone, the blood glucose level can be improved, and as a result, new abnormal stem cells are no longer formed, and the diabetes mellitus can be cured.

Therefore, in one embodiment, the blood glucose in the treatment and/or prevention of diabetes mellitus and/or a disease, disorder, and/or symptom associated with diabetes mellitus of the present disclosure can be controlled due to HDAC regulation, and a subject whose blood glucose is expected to be reduced by the HDAC regulation (such as a subject with type 2 diabetes mellitus whose blood glucose level is not significantly high) may be preferably treated.

In addition to removal of abnormal stem cells, HDAC regulation can also suppress various cells derived from abnormal stem cells (vascular endothelial cells and cells having abnormal cell fusion ability). It can be advantageous to use an HDAC regulator that acts not only on stem cells but also on cells derived therefrom, such as particularly in the treatment of advanced diabetes mellitus, and/or to further use another drug that suppresses induced cells.

### (Characteristics of abnormal stem cells)

Abnormal stem cells that can be associated with diabetes mellitus have been found, and the characteristics of the abnormal stem cells are described below. In one embodiment, the abnormal stem cell can be a hematopoietic stem cell. In one embodiment, the abnormal stem cells are characterized by not expressing and/or functioning at least one of CD106, histone deacetylases (HDACs), CD34, TNF-α, and proinsulin at a normal level, and if necessary, may be further characterized by having other characteristics of the abnormal stem cells described in the present specification (for example, c-Kit positive, Sca-1 positive, hematopoietic stem cell lineage marker negative, and short-term hematopoietic stem cell characteristics (such as CD38 negative and the like)).

In one embodiment, the abnormal stem cell can be characterized by an expression level of CD106 that is higher than an expression level of CD106 in total bone marrow cells or hematopoietic stem cells (for example, CD34 positive and Thy-1 positive cells) of a population of non-diabetic subjects. In one embodiment, the abnormal stem cell can be characterized by an mRNA expression level of CD106 that is higher than that of hematopoietic stem cells (for example, CD34 positive cells) derived from a population of non-diabetic subjects by 10% or more, 20% or more, 30% or more, 40% or more, 50% or more, 60% or more, 70% or more, 80% or more, 90% or more, 100% or more, 150% or more, or 200% or more.

In one embodiment, the abnormal stem cells may be characterized by expression of histone deacetylase genes (HDACs). In one embodiment, the abnormal stem cell can be characterized by an expression level of one or more of histone deacetylase genes (HDACs) (for example, HDAC 2, 3, 4, 8, 9, 11, SIRT1, 6) that is higher or lower than that of hematopoietic stem cells (for example, c-Kit positive lineage marker-negative cells) from a population of non-diabetic subjects by 10% or more, 20% or more, 30% or more, 40% or more, 50% or more, 60% or more, 70% or more, 80% or more, 90% or more, 100% or more, 150% or more, 200% or more, 300% or more, 400% or more, or 500% or more.

In one embodiment, the abnormal stem cell can be characterized by expression of TNF-α. In one embodiment, the abnormal stem cell can be characterized by being positive for expression of TNF-α. In one embodiment, the abnormal stem cell can be characterized by an mRNA expression level of TNF-α that is higher than that of hematopoietic stem cells (for example, CD34 positive cells) derived from a population of non-diabetic subjects by 10% or more, 20% or more, 30% or more, 40% or more, 50% or more, 60% or more, 70% or more, 80% or more, 90% or more, 100% or more, 150% or more, or 200% or more.

In one embodiment, the abnormal stem cell can be characterized by abnormal expression of proinsulin. In one embodiment, the abnormal stem cell can be characterized by being positive for expression of proinsulin. In one embodiment, the abnormal stem cell can be characterized by an expression level of mRNA of insulin (or proinsulin) that is higher than that of hematopoietic stem cells (for example, CD34 positive cells) derived from a population of non-diabetic subjects.

In one embodiment, the abnormal stem cell can be characterized by expression of one or more of c-Kit, Sca-1, and a hematopoietic stem cell lineage marker. In one embodiment, the abnormal stem cell can be characterized by being positive for expression of one or more of c-Kit and Sca-1. Examples of the hematopoietic stem cell lineage marker include CD3 (T cell), CD19 (B cell), NK1.1 (NK cell), CD11c (dendritic cell), CD11b (monocyte), FcεRI (mast cell), and Gr-1 (granulocyte). In one embodiment, the abnormal stem cell can be characterized by being negative for expression of one or more (for example, all) of the hematopoietic stem cell lineage markers. In one embodiment, the abnormal stem cell can be characterized by an expression level of mRNA of CD34 that is higher than that of hematopoietic stem cells derived from a population of non-diabetic subjects.

In one embodiment, the abnormal stem cell can be characterized by being a hematopoietic stem cell. In one embodiment, being human hematopoietic stem cells can also be characterized by being CD34 positive and Thy-1 positive or being Lineage negative, CD34 positive, CD38 negative, CD90 positive, and CD45RA negative. In one embodiment, the abnormal stem cell can be characterized by being a cell stage of a hematopoietic stem cell. In one embodiment, the abnormal stem cell can be a short-term hematopoietic stem cell.

In one embodiment, determination that a subject has an abnormal stem cell can be performed by detecting any characteristic of the abnormal stem cell described in the present specification. In one embodiment, whether the subject has an abnormal stem cell can be diagnosed based on a quantitative index of at least one characteristic of the abnormal stem cell described in the present specification (for example, a cell surface protein expression level and an mRNA expression level). In one embodiment, in a case where a sample (for example, a blood sample or a bone marrow sample) derived from the subject or a hematopoietic stem cell (for example, a CD34 positive cell) contained in the sample exhibits an expression level of mRNA of an abnormal stem cell marker (CD106, HDAC, TNF-α, or the like) that is higher than that of a corresponding sample of a population of non-diabetic subjects or a hematopoietic stem cell (for example, a CD34 positive cell) contained in the sample by 10% or more, 20% or more, 30% or more, 40% or more, 50% or more, 60% or more, 70% or more, 80% or more, 90% or more, 100% or more, 150% or more, or 200% or more, a subject can be determined to be suitable for the treatment and/or prevention of diabetes mellitus and/or a disease, disorder, and/or symptom associated with diabetes mellitus of the present disclosure.

In one embodiment, determination that a subject has an abnormal stem cell can be performed based on a percentage of cells exhibiting at least one characteristic (including a quantitative indicator) of the abnormal stem cells described in the present specification. In one embodiment, the diagnosis of the present disclosure can be performed based on a percentage of cells exhibiting expression of an abnormal stem cell marker (CD106, HDAC, TNF-α, or the like) at a level that is not normal among hematopoietic stem cells of a subject, and if necessary, the diagnosis of the present disclosure can be performed by comparison with a percentage of cells exhibiting CD106 expression at a level that is not normal among hematopoietic stem cells in a population of non-diabetic subjects or a population of a diabetic subjects. In one embodiment, a subject can be determined to be suitable for the treatment and/or prevention of diabetes mellitus and/or a disease, disorder, and/or symptom associated with diabetes mellitus of the present disclosure when a percentage of cells exhibiting expression of an abnormal stem cell marker (CD106, HDAC, TNF-α, or the like) at a level that is not normal among hematopoietic stem cells of a subject in a particular site (for example, bone marrow) is 1. 1-fold or more, 1.2-fold or more, 1.3-fold or more, 1.4-fold or more, 1.5-fold or more, 1.6-fold or more, 1.7-fold or more, 1.8-fold or more, 1.9-fold or more, 2-fold or more, 2. 1-fold or more, 2.2-fold or more, 2.3-fold or more, 2.4-fold or more, 2.5-fold or more, 2.6-fold or more, 2.7-fold or more, 2.8-fold or more, 2.9-fold or more, 3-fold or more, 3.5-fold or more, 4-fold or more, 4.5-fold or more, or 5-fold or more of a percentage of cells exhibiting expression of an abnormal stem cell marker (CD106, HDAC, TNF-α, or the like) at a level that is not normal among hematopoietic stem cells in a population of non-diabetic subjects.

In one embodiment, the subject to whom the treatment and/or prevention of the present disclosure is applied is diagnosed with diabetes mellitus. In one embodiment, the subject to whom the treatment and/or prevention of the present disclosure is applied is diagnosed with, or is at risk for, complications of diabetes mellitus, for example, a neurological disorder, nephropathy, a hepatic disorder, retinopathy, fatty liver, a gastrointestinal disorder, delayed fracture healing, an eating disorder, and a skin disorder. In one embodiment, the subject to whom the treatment and/or prevention of the present disclosure has a thymus (for example, has not undergone thymectomy). In one embodiment, the subject to whom the treatment and/or prevention of the present disclosure does not have a decreased thymic function. In one embodiment, the treatment and/or prevention of the present disclosure includes administration of a drug that enhances a thymic function.

As described as used herein, since the present inventors have found that abnormal hematopoietic stem cells can cause diabetes mellitus and/or a disease, disorder, and/or symptom associated with diabetes mellitus, a companion treatment for treating diabetes mellitus and/or a disease, disorder, and/or symptom associated with diabetes mellitus based on detection of abnormal hematopoietic stem cells is one of the preferred embodiments contemplated in the present disclosure. Similarly, the present disclosure may be used with a companion diagnostic drug containing a drug that detects abnormal hematopoietic stem cells to lead the treatment or prevention of diabetes mellitus and/or a disease, disorder, and/or symptom associated with diabetes mellitus. Although details of methods and drugs for detecting abnormal hematopoietic stem cells are detailed elsewhere as used herein, a subject in which abnormal hematopoietic stem cells are detected in such a manner can be a subject to whom any of the treatment or prevention of diabetes mellitus and/or a disease, disorder, and/or symptom associated with diabetes mellitus described in the present specification is suitably administered. Details of the treatment or prevention can be appropriately adjusted according to the detection level of abnormal hematopoietic stem cells, the detection site, and other conditions of the subject (diabetes mellitus progression level, age, gender, and the like). Thus, the detection of abnormal hematopoietic stem cells can be an important factor in determining the strategy for the treatment or prevention of diabetes mellitus and/or a disease, disorder, and/or symptom associated with diabetes mellitus.

The treatment, prevention, and/or diagnosis of the present disclosure can be performed in any subject. In one embodiment, the subject is a mammal, for example, a human, a mouse, a guinea pig, a hamster, a rat, a mouse, a rabbit, a pig, a sheep, a goat, a cow, a horse, a cat, a dog, a marmoset, a monkey, or a chimpanzee. In specific embodiments, the subject is a human.

### (Medicine, dosage form, and the like)

The HDAC regulator and hypoglycemic agent described in the present specification can be provided as a composition or medicine in various forms.

An administration route of the HDAC regulator and hypoglycemic agent described in the present specification is preferably an administration route that is effective in the treatment or prevention of diabetes mellitus and/or a disease, disorder, and/or symptom associated with diabetes mellitus, and may be, for example, intravenous, subcutaneous, intramuscular, intraperitoneal, or oral administration. The administration form may be, for example, an injection, a capsule, a tablet, a granule, or the like. An aqueous solution for injection may be stored, for example, in a vial or in a stainless container. In addition, the aqueous solution for injection may contain, for example, physiological saline, sugar (for example, trehalose), NaCl, NaOH, or the like. In addition, the therapeutic drug may be formulated with, for example, a buffer (for example, a phosphate buffer), a stabilizer, or the like.

In general, the composition, medicine, suppressing agent, migration agent, detection agent, or the like of the present disclosure contains a therapeutically effective amount of active ingredient or a detectable amount of detection means, and a pharmaceutically acceptable carrier or excipient. As used herein, the term "pharmaceutically acceptable" means government regulatory agency-approved or pharmacopoeia or other commonly recognized pharmacopoeia-listed for use in animals and more specifically in humans. The term "carrier" used in the present specification refers to a diluent, adjuvant, excipient, or vehicle administered in conjunction with a therapeutic agent or detection agent. Such a carrier can be an aseptic liquid, for example, water or oil, includes a carrier derived from petroleum, an animal, a plant, or a synthetic origin, and includes, but is not limited to, peanut oil, soybean oil, mineral oil, sesame oil, and the like. When a medicine is orally administered, water is a preferred carrier. When the pharmaceutical composition is administered intravenously, saline and aqueous dextrose are preferred carriers. Preferably, an aqueous saline solution, and aqueous dextrose and a glycerol solution are used as a liquid carrier for an injectable solution. Examples of a preferred excipient include light anhydrous silicic acid, crystalline cellulose, mannitol, starch, glucose, lactose, sucrose, gelatin, malt, rice, wheat flour, chalk, silica gel, sodium stearate, glycerol monostearate, talc, sodium chloride, powdered skim milk, glycerol, propylene, glycol, water, ethanol, carmellose calcium, carmellose sodium, hydroxypropyl cellulose, hydroxypropyl methylcellulose, polyvinyl acetal diethylamino acetate, polyvinylpyrrolidone, gelatin, medium-chain fatty acid triglyceride, polyoxyethylene hydrogenated castor oil 60, saccharose, carboxymethylcellulose, corn starch, and an inorganic salt. The composition can contain a small amount of a wetting agent or an emulsifier, or a pH buffer, if desired. These compositions can be in the form of a solution, suspension, emulsion, tablet, pill, capsule, powder, sustained release mixture, or the like. The composition can also be formulated as a suppository using a traditional binding agent and carrier, for example, triglyceride. Oral formulation can also contain a standard carrier such as medicine grade mannitol, lactose, starch, magnesium stearate, sodium saccharin, cellulose, or magnesium carbonate. Examples of a preferred carrier are described in E. W. Martin, Remington's Pharmaceutical Sciences (Mark Publishing Company, Easton, U.S.A). Such a composition contains a therapeutically effective amount of a therapeutic agent, and preferably in a purified form, together with a preferred amount of carrier, such that the composition is provided in a form preferred for administration to a patient. The formulation should be preferable for the mode of administration. In addition, for example, a surfactant, an excipient, a coloring agent, a flavoring agent, a preservative, a stabilizer, a buffer, a suspension, an isotonizing agent, a binder, a disintegrant, a lubricant, a fluidity accelerator, a corrigent, or the like may be contained.

Examples of the "salt" in one embodiment of the present disclosure include an anionic salt formed with any acidic (for example, carboxyl) group and a cationic salt formed with any basic (for example, amino) group. Salts include an inorganic salt and an organic salt, as well as the salt described in, for example, Berge et al., J.Pharm. Sci., 1977, 66, 1-19. In addition, examples thereof include a metal salt, an ammonium salt, a salt with an organic base, a salt with an inorganic acid, and a salt with an organic acid. The "solvent" in one embodiment of the present disclosure is a compound formed with a solute and a solvent. For example, J. Honig et al., The Van Nostrand Chemist's Dictionary P650 (1953) can be referred for the solvate. When a solvent is water, the solvate formed is a hydrate. It is preferable that the solvent does not interfere with the biological activity of the solute. Examples of such a preferred solvent include, but are not particularly limited to, water and various buffers.

In the present disclosure, when a medicine is administered, various delivery systems can be used together, and such systems can be used to administer the suppressing agent and/or migration agent of the present disclosure to an appropriate site. Such a system includes, for example, liposomes, microparticles, and encapsulation in microcapsules; use of endocytosis mediated by a receptor; and construction of a therapeutic nucleic acid as a part of a retroviral vector or other vectors. The method of introduction includes, but is not limited to, intradermal, intramuscular, intraperitoneal, intravenous, subcutaneous, intranasal, epidural, and oral routes. It is also possible to administer the medicine by any suitable route, for example, by injection, bolus injection, or absorption through epithelial or mucocutaneous lining (for example, oral cavity, rectum, intestinal mucosa, or the like). In addition, an inhaler or nebulizer using an aerosolizing agent can be used as necessary. In addition, other biological active agents can also be administered together. The administration can also be systemic or local.

In a preferred embodiment, a composition can be formulated as a pharmaceutical composition adapted for administration to humans according to a known method. Such a composition can be administered by injection. A composition for use in injection administration is typically a solution in an aseptic isotonic aqueous buffer. The composition can also contain a solubilizing agent and a local anesthetic agent such as lidocaine which alleviates the pain at the site of injection as necessary. In general, the ingredients can be supplied separately or mixed and supplied together in a unit dosage form, and supplied as a lyophilized powder or water free concentrate, for example, in a sealed container such as an ampoule or sachet showing the amount of active agent. When the composition is to be administered by injection, the composition can be distributed by using an injection bottle containing aseptic agent-grade water or saline. When the composition is to be administered by injection, aseptic water or saline ampoule for injection can also be provided so that the ingredients can be mixed prior to administration.

The HDAC regulator and hypoglycemic agent of the present disclosure can be formulated as a neutral or salt form or other prodrugs (for example, esters or the like). A pharmaceutically acceptable salt includes a salt formed with a free carboxyl group derived from hydrochloric acid, phosphoric acid, acetic acid, oxalic acid, tartaric acid, or the like, a salt formed with a free amine group derived from isopropylamine, triethylamine, 2-ethylaminoethanol, histidine, procaine, or the like, and a salt derived from sodium, potassium, ammonium, calcium, ferric hydroxide, or the like.

The amount of the HDAC regulator and hypoglycemic agent of the present disclosure can vary depending on the properties of the disorder or state, and can be determined by those skilled in the art by a standard clinical technique based on the descriptions in the present specification. Furthermore, an in vitro assay can be used in some cases to assist the identification of the optimal dosing range. The precise dose to be used in a formulation can also vary depending on the administration route or the severity of the disease or disorder. Thus, the dose should be determined in accordance with the judgment of the attending physician or the condition of each patient. However, the dosage is not particularly limited, and may be, for example, 0.001, 1, 5, 10, 15, 100, or 1,000 mg/kg body weight per administration, or may be within a range of any two of these values. The dosing interval is not particularly limited, and may be, for example, 1 or 2 administrations every 1, 7, 14, 21, or 28 days or 1 or 2 administrations per range of any two of these values. The dosage, dosing interval, and dosing method may be appropriately selected depending on the age, weight, symptom, target organ, or the like of the patient. In addition, it is preferable that a therapeutic drug contains a therapeutically effective amount or an effective amount of an active ingredient that exerts a desired action. The effective dose can be estimated from a dose-response curve obtained from in vitro or animal model testing systems.

The pharmaceutical composition, therapeutic agent, or prophylactic agent of the present disclosure can be provided as a kit.

In a specific embodiment, the present disclosure provides a drug pack or kit including one or more containers filled with one or more components of the composition or medicine of the present disclosure. In some cases, information indicating approval of manufacture, use, or sale for administration to humans by a government agency regulating the manufacture, use, or sale of medicines or biological products in a stipulated form can be appended to such a container.

The formulation procedure for the therapeutic drug, prophylactic drug, or the like of the present disclosure as a medicine or the like is known in the art, and is described, for example, in the Japanese Pharmacopoeia, the United States Pharmacopeia, pharmacopeia of other countries, or the like. Accordingly, those skilled in the art can determine the embodiment, such as the amount to be used, without undue experimentation from the descriptions in the present specification.

As used herein, "or" is used when "at least one or more" of the items listed in the text can be employed. The same applies to "or". When explicitly described in the present specification as "within the range" of "two values", the range also includes the two values themselves.

Reference literatures such as scientific literatures, patents, and patent applications cited in the present specification are incorporated herein by reference to the same extent that the entirety of each document is specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described as used herein, but is limited only by the claims.

### Examples

If necessary, an animal used in the following Example is handled in accordance with the animal experiments guidelines of Shiga University of Medical Science. For reagents, the specific products described in the Examples were used. However, the reagent can be substituted with an equivalent product from another manufacturer (Sigma-Aldrich, Wako Pure Chemical, Nacalai Tesque, R&D Systems, USCN Life Science INC, or the like).

In the following Examples, the abbreviations each have the following meaning.
- DM: Diabetes mellitus
- nonDM: Non-diabetes mellitus
- STZ-DM: Streptozotocin-induced diabetes mellitus

### (Example 1: Bone marrow transplantation treatment of STZ-DM mice and HDAC expression)

Diabetes mellitus in STZ-DM mice (type 2 diabetes mellitus model) was treated by bone marrow transplantation. In addition, HDAC expression was also compared between non-diabetes mellitus and diabetes mellitus. In STZ-DM mice, unlike non-DM mice, it was found that TNF-α positive cells and proinsulin positive cells were present and CD106 expressing cells were increased in c-kit positive Sca-1 positive lineage marker-negative (KSL) cells. In addition, it was found that TNF-α positive cells and CD106 expressing cells were increased also in KSL cells of type 1 diabetic mice (NOD mice), and it was considered that hematopoietic stem cells having these characteristics caused appearance of characteristics as a chronic disease of diabetes mellitus.

In addition, it was reported that when bone marrow cells stained with Hoechest 33342 dye were excited with ultraviolet light (350 nm) and developed with two types of optical filters, Hoechst blue and Hoechst red, a hematopoietic stem cell fraction was included in an unstained side population (SP cells) (see J Exp Med. 1996 Apr 1; 183(4): 1797-806; Nat Med. 1997 Dec; 3(12): 1337-45). A test was performed using non-DM mice and STZ-DM mice based on this staining, and it was found that CD106 positive cells and TNF-α positive cells characterizing hematopoietic stem cell abnormalities were included in non-SP cells (short-term hematopoietic stem cells: ST-HSCs) in KSL cells, but not in SP cells (long-term hematopoietic stem cells: LT-HSCs) in KSL cells at an earlier differentiation stage, and abnormal stem cells found in diabetes mellitus were present in ST-HSCs. Expression of a histone deacetylase (HDAC) gene was examined in abnormal stem cells found to be involved in diabetes mellitus as described above.

An overview of the procedure is illustrated in FIG. 4. Diabetes mellitus was induced by tail vein injection of streptozotocin (150 mg/kg) into C57BL/6J mice (wild type, CLEA Japan, Inc., Osaka), and mice having a blood glucose level of 250 mg/dl or higher at 1 week after administration were used as diabetic mice (STZ-DM mice). Four weeks after the onset of diabetes mellitus, an insulin pellet (insulin 0.1 U/day release, sustained for approximately 30 days, LINSHIN CANADA, INC., Cat No: Pr-1-B) or a blank pellet (without insulin) was subcutaneously inserted into the back of the neck of each mouse. On the next day, the mice were irradiated with a lethal dose of radiation (9 Gy), and the mice were transplanted with total bone marrow cells (4 × 10⁶) obtained from non-diabetic patients at 12 weeks of diabetes mellitus or the same age, and the progress was observed for 8 weeks. Meanwhile, blood glucose levels were measured weekly and the treatment effect was observed. Mice were euthanized after the end of the treatment period and histological analysis was performed. In addition, for the bone marrow cells used for bone marrow transplantation, a hematopoietic stem cell fraction (KSL) was collected, and the expressed genes were subjected to microarray analysis.

Preparation of sections for histological analysis was performed as follows. Mice were subjected to perfusion fixation with a 4% PFA solution in 0.1 M PB and harvested pancreatic tissue was immersed overnight in a 15% sucrose solution in 0.1 M PB, and then, 8 um frozen sections were prepared with a cryostat. The frozen sections were washed 3 times with PBS (-) for 10 minutes, reacted in a 0.3% hydrogen peroxide solution (0.3% H₂O₂ in PBS (-)) for 30 minutes at room temperature to inactivate endogenous peroxidase in the sections, and then washed 3 times for 5 minutes with PBS (-). Thereafter, in order to prevent non-specific reactions, the sections were reacted with 5% normal goat serum in PBS (-) supplemented with 0.3% Triton X and blocked at room temperature for 1 hour. After blocking, the sections were reacted with a primary antibody (anti-insulin antibody: CST Inc. or anti-glucagon antibody: CST Inc.) at 4°C overnight. Thereafter, the sections was washed 3 times with PBS (-) for 5 minutes, reacted in ImmPRESS reagent (Vector laboratory) at room temperature for 30 minutes, and further washed. Thereafter, DAB color development was performed, and nuclear staining was performed with hematoxylin as counterstaining, and then, dehydration and encapsulation were performed. The prepared sections were observed under a microscope, about 5 to 8 pancreatic islets were randomly selected, and the percentage of insulin positive cells relative to the size of the pancreatic islets was calculated using Image J software. In addition, mice in each experimental group were subjected to perfusion fixation, and then dissected to collect the thymus. An image of the thymus (either left lobe or right lobe) was acquired under a stereomicroscope.

Microarray analysis was performed as follows. Mononuclear cells were isolated by FACS from whole bone marrow using Ficoll-Paque Plus (GE Healthcare Bio-Sciences AB, Uppsala, Sweden). The mononuclear cells were reacted with PE-Cy7-conjugated streptavidin antibodies, APC-conjugated anti-c-kit antibodies, APC-Cy7-conjugated anti-Ly6A/E antibodies (Sca-1), and a biotin mouse lineage panel, and then fixed with BD Cytofix/CytoPerm (BD Biosciences). Next, rabbit anti-insulin monoclonal antibodies (Cell signaling technology) and PE-conjugated anti-rabbit IgG antibodies (Cell signaling technology) were applied to mononuclear cells. Prior to the staining, LIVE/DEAD fixable dead cell blue staining kit (ThermoFisher Scientific Inc., Waltham, MA, USA) was used to deplete dead cells. RNA was extracted from c-kit positive and Sca-1 positive lineage marker-negative (KSL) cells in the mononuclear cells obtained by FACS, and gene expression of a histone deacetylase gene group (Hdacs) was analyzed by microarray analysis. In the microarray analysis, RNA was isolated from LSK cells using RNeasy Plus Micro kit (Qiagen, Tokyo, Japan). An RNA sample was analyzed on TaKaRa bio (Japan, Shiga) using Nanodrop and Agilent 2100 BioAnalysers. The RNA sample was amplified on Ovation Pico WTA system and used for microarray analysis. A large number of isozymes in HDAC9 and the like were present, but in the microarray, an array targeting a location reflecting the overall amount was used.

### Results

The results of expression comparison of histone deacetylases (Hdac1 to 11 and Sirtuin1 to 7) in the hematopoietic stem cell fraction of diabetic and non-diabetic bone marrow are illustrated in FIG. 5. In diabetes mellitus, Hdac3, Hdac4, Hdac8, Sirt1, and Sirt6 were increased. In addition, Hdac2, Hdac9, and Hdac11 were decreased. In consideration of a balance with the effect on diabetes mellitus as a whole, it is considered preferable to regulate HDAC so as to suppress abnormal hematopoietic stem cells, and for example, a strategy of broadly inhibiting class I, II, and IV HDACs without affecting class III HDAC is presumed.

In diabetic KSL cells, the expression of epigenome-related genes (Hdacs) important for regulating gene expression by histone modification or the like was increased, and thus, it was considered that hyperglycemia imparted abnormal properties to stem cells at the gene level. It was reported that the abnormality of cells generated by hyperglycemia was maintained even when cells exposed to transient hyperglycemia were returned to the environment of normoglycemia (El-Osta et. al. J Exp Med. 2008 Sep 29; 205 (10): 2409-17), and it was also considered from this that the increased expression of histone deacetylase gene generated in KSL cells was involved in the appearance of abnormal stem cells and diabetes mellitus.

The transition of the blood glucose level over time is illustrated in FIG. 6. In mice not treated with insulin, hyperglycemia persisted even when diabetic bone marrow was normalized by bone marrow transplantation. It is estimated from this that new abnormal hematopoietic stem cells are generated due to hyperglycemia regardless of how much the bone marrow is normalized in a state in which blood glucose is not normalized. On the other hand, when the bone marrow stem cells were normalized simultaneously with the blood glucose control for 4 weeks by the insulin pellet, the hyperglycemia was corrected. However, in mice transplanted with bone marrow obtained from diabetic mice, the blood glucose level rapidly increased when the insulin action was lost. This shows that abnormal pathological stem cells are present in the bone marrow of the diabetic subject.

As a result of insulin DAB staining of the pancreas (FIG. 7), it was suggested that in the non-diabetic bone marrow transplantation group to which the insulin pellet was administered, the percentage of insulin positive cells was increased as compared with the STZ diabetic group, and insulin production was improved. None of the non-diabetic bone marrow transplantation group to which the blank pellet was administered, the diabetic bone marrow transplantation group to which the insulin pellet was administered, and the diabetic bone marrow transplantation group to which the blank pellet was administered showed improvement in insulin production.

In rodents, glucagon is found around 10% of the pancreatic islets, but in the diabetic state, glucagon significantly increases to occupy approximately 70% of the center of the pancreatic islets. As a result of pancreatic glucagon DAB staining (FIG. 8), in the non-diabetic bone marrow transplantation group to which the insulin pellet was administered, the percentage of glucagon positive cells was decreased and glucagon production significantly decreased compared to the STZ diabetic group. In all cases of the non-diabetic bone marrow transplantation group to which the blank pellet was administered, the diabetic bone marrow transplantation group to which the insulin pellet was administered, and the diabetic bone marrow transplantation group to which the blank pellet, the percentage of glucagon positive cells was similar to that of STZ diabetes mellitus, and no decrease in glucagon production was observed. When the size of the collected thymus was compared (FIG. 9), the thymus was significantly atrophic in diabetic mice compared with non-diabetic mice. On the other hand, when the insulin pellet was administered and the bone marrow of the non-diabetic mouse was transplanted, the size of the thymus recovered as in the non-diabetic mouse, and no improvement was observed in other mice.

### (Example 2: Tissue analysis using GFP transgenic (GFP-TG) mice)

As illustrated in FIG. 10, histological analysis was performed on diabetes mellitus using bone marrow cells of GFP-TG mice.

Specifically, normal mice were transplanted with total bone marrow cells (4 × 10⁶) obtained from non-diabetic GFP-TG mice, and then observed for 4 weeks. After fixing of the transplanted bone marrow, STZ was administered to develop diabetes mellitus. A buffer was administered to the non-diabetic group. Both groups were euthanized 12 weeks after diabetes mellitus was stabilized and histological analysis was performed.

The tissue analysis of the treatment study using bone marrow transplantation obtained from GFP-TG mice was performed as follows. Normal mice were administered with STZ to develop diabetes mellitus and were observed for 4 weeks. Thereafter, mice were divided into two groups and implanted with an insulin pellet in one group and a blank pellet in the other. On the next day, the mice were irradiated with a lethal dose of radiation (9 Gy), total bone marrow cells (4 × 10⁶) obtained from GFP-TG normal mice were transplanted into both groups, and the progress was observed for 8 weeks. Meanwhile, blood glucose levels were measured weekly and the treatment effect was observed. Mice were euthanized after the end of the treatment period, and the pancreatic islets and thymus were histologically analyzed. For immunohistological analysis, specific antibodies of TNF-α and Vcam were observed with a fluorescence microscope.

### Results

FIG. 11 illustrates the result of analyzing pancreatic islets. In non-diabetic mice, bone marrow-derived cells were slightly found in the pancreatic islets, whereas Vcam1 positive cells were found only in a part of the outer membrane of the pancreatic islet and were hardly found within the pancreatic islets. On the other hand, in diabetic pancreatic islets, the number of bone marrow-derived cells increased, and at the same time, a large number of Vcam1 positive cells were observed. Furthermore, in some cells, Vcam1 positive cells (red) and bone marrow-derived cells (green) were overlapped, and yellow cells were observed. This suggests that some of bone marrow-derived cells have cells expressing Vcam1 in pancreatic islets. On the other hand, when diabetic mice were normalized to blood glucose by the insulin pellet and non-diabetic bone marrow was transplanted, almost the same findings as those of non-diabetic mice were obtained in pancreatic islets, whereas when the blood glucose was not normalized, the same findings as those of untreated diabetic mice were obtained. This indicates that even when the bone marrow of a non-diabetic mouse is transplanted to replace the bone marrow while the diabetic mouse remains in a hyperglycemic state, abnormal cells positive for Vcam1 are generated again due to hyperglycemia, and the abnormal cells enter the pancreatic islets.

FIG. 12 illustrates findings of the thymus. Green cells appeared significantly in thymic tissue in the mice transplanted with bone marrow obtained from non-diabetic GFP mice (GFP BMT nonDM) after irradiation with 9 Gy radiation compared to the non-diabetic mice. It is considered that this occurred because thymocytes injured by irradiation were regenerated by bone marrow-derived cells. However, when STZ is administered to the mouse to develop diabetes mellitus, most of thymocytes derived from bone marrow are injured and eliminated due to hyperglycemia, and again, marked atrophy is shown (GFP BMT STZDM). On the other hand, even when a diabetic mouse is irradiated with radiation and bone marrow of a non-diabetic mouse is transplanted thereto, atrophic thymocytes are not regenerated (blank pellet + nonDM GFP BMT). However, when glycemic control is improved, the thymus is regenerated by bone marrow-derived cells (insulin pellet + nonDM GFP BMT).

### (Example 3: Drug treatment of STZ-DM mice)

An HDAC regulator and blood glucose control were combined to treat diabetes mellitus in STZ-DM mice.

An overview of the procedure is illustrated in FIG. 13. Streptozotocin (150 mg/kg) was tail vein-administered to C57BL/6 J mice (wild type, CLEA Japan, Inc., Osaka) (nonDM) to develop diabetes mellitus. For the STZ-DM mouse, the blood glucose level was normalized by insulin pellet administration, and the blank pellet was administered to a control group. In both the treatment group and the control group, 2.5 mg/kg of givinostat (Cayman chemical, USA) was administered to the tail vein or orally. The insulin pellet or the blank pellet was administered up to 3 days before the start of the givinostat treatment, and the administration of givinostat was started on the day on which it was confirmed that the blood glucose level of the treatment group fell within the normal range on day 0. Note that givinostat was administered once every three days until week 8. In addition, a non-diabetic group (nonDM) in which only a buffer was administered without administering STZ simultaneously with the treatment group, and a non-treated diabetic group (STZ-DM) in which neither blood glucose control nor treatment with givinostat was performed were also observed simultaneously.

Blood glucose levels were measured weekly for 12 weeks. In addition, an intraperitoneal glucose tolerance test (IPGTT) was performed on some mice 12 weeks after the start of treatment. After 16 hours of fasting, blood glucose level measurement and blood collection were performed at 0 minutes, 100 µl of a 1.5 g/kg glucose solution was intraperitoneally administered, and blood glucose level measurement and blood collection were performed at 30 minutes, 60 minutes, and 120 minutes, respectively. Serum was collected from the collected blood, and the serum insulin concentration was measured using an ultra-sensitive mouse insulin measurement kit (Morinaga Institute of Biological Science, Inc., Japan). FIG. 15 illustrates the transition of blood glucose, and FIG. 16 illustrates the transition of insulin concentration.

12 weeks after the start of treatment, some mice were subjected to perfusion fixation with a 4% PFA solution in 0.1 M PB and harvested pancreatic tissue was immersed overnight in a 15% sucrose solution in 0.1 M PB, and then, 8 um frozen sections were prepared with a cryostat. The frozen sections were washed 3 times with PBS (-) for 10 minutes, reacted in a 0.3% hydrogen peroxide solution (0.3% H₂O₂ in PBS (-)) for 30 minutes at room temperature to inactivate endogenous peroxidase in the sections, and then washed 3 times for 5 minutes with PBS (-). Thereafter, in order to prevent non-specific reactions, the sections were reacted with 5% normal goat serum in PBS (-) supplemented with 0.3% Triton X and blocked at room temperature for 1 hour. After blocking, the sections were reacted with a primary antibody (anti-insulin antibody: CST Inc. or anti-glucagon antibody: CST Inc.) at 4°C overnight. Thereafter, the sections was washed 3 times with PBS (-) for 5 minutes, reacted in ImmPRESS reagent (Vector laboratory) at room temperature for 30 minutes, and further washed. Thereafter, DAB color development was performed, and nuclear staining was performed with hematoxylin as counterstaining, and then, dehydration and encapsulation were performed. The prepared sections were observed under a microscope, about 5 to 8 pancreatic islets were randomly selected, and the percentage of insulin or glucagon positive cells relative to the size of the pancreatic islets was calculated using Image J software.

In addition, mice in each experimental group were subjected to perfusion fixation, and then dissected to collect the femur, thymus, and pancreas. In the femur, bone tissue was demineralized using a neutral demineralizing solution (10% EDTA solution) to prepare a paraffin block. A paraffin section having a thickness of 3 µm was prepared from the paraffin block using a microtome, and was stained with DAB. As pretreatment for DAB staining, the paraffin section was deparaffinized with xylene and hydrophilized with ethanol, and the antigen was activated using HistoVT one (Nacalai Tesque). Thereafter, DAB staining was performed using TNF-α (abcam).

### Results

FIG. 14 illustrates changes in blood glucose levels of each group. Compared to nonDM, hyperglycemia persisted in STZ-DM. On the other hand, the blood glucose levels immediately decreased in the diabetic group in which the blood glucose control was performed with the insulin pellet for about 4 weeks and the treatment group in which givinostat (oral or intravenous infusion for 8 weeks) was administered simultaneously with the insulin pellet. However, in the group not treated with the insulin pellet, the blood glucose level did not decrease even when givinostat was administered for 8 weeks, and the increase in blood glucose was not suppressed in all the periods up to 12 weeks after the discontinuation of givinostat. In addition, in the group in which only the insulin pellet was used and givinostat was not administered, the blood glucose level gradually increased from week 2 after the start of the insulin pellet, and after week 5, hyperglycemia occurred as in the group in which blood glucose control was not performed. Note that, in this group, since the general condition of some mice was not good after the start of treatment, the mice were euthanized at week 9. In contrast to these groups, in the group in which givinostat was administered simultaneously with blood glucose control (both orally and intravenously), the reduction in blood glucose level continued even after discontinuing givinostat after week 9.

Responsiveness in an intraperitoneal glucose tolerance test (IPGTT) was examined between the mice treated with the insulin pellet + givinostat and the mice treated with the blank pellet + givinostat (FIGS. 15 and 16), and early normalization of blood glucose levels and high blood insulin elevation were observed in the mice treated with the insulin pellet + givinostat, although the release of insulin from the insulin pellet had already ended. It is considered from this that diabetes mellitus is cured and the pancreatic function is restored in the mice treated with insulin pellet + givinostat.

As a result of insulin DAB staining of the pancreas (FIG. 17), compared with STZ-DM, in the group in which givinostat was administered simultaneously with blood glucose control, the area of insulin positive cells significantly increased, and recovered to about 1/2 of nonDM. However, in all the groups in which blood glucose did not normalize, the insulin area did not differ from STZ-DM. Interestingly, the insulin positive cells decreased overall in the group with poor blood glucose control, whereas the insulin positive cells recovered mainly in the center of the pancreatic islets in the group with good blood glucose control.

As a result of glucagon DAB staining of the pancreas (FIG. 18), in NonDM, glucagon positive cells existed so as to draw a circle immediately below the outer membrane of the pancreatic islets, and about 10% of the entire pancreatic islets were positive for glucagon positive cells. In contrast, in STZ-DM, glucagon positive cells remarkably increased in the entire pancreatic islets, occupying 75% of the entire pancreatic islets. In comparison, in the group to which givinostat was administered simultaneously with blood glucose control, the area of glucagon positive cells was reduced from the center to about 40% of the entire pancreatic islets. On the other hand, in the group showing hyperglycemia, the glucagon positive area remained high.

When the weights of the collected thymus were compared (FIG. 19), the thymus weight was 37 mg in the non-diabetic group, but decreased to 13 mg and approximately 1/3 in the diabetic group. In the group treated with givinostat simultaneously with blood glucose control, both oral and intravenous administration recovered to about 2/3 of normal.

When bone marrow and thymus were subjected to TNF-α immunostaining (DAB staining) (FIGS. 20 and 21), TNF-α positive cells increased in the diabetic group, but TNF-α positive cells decreased to the same extent as the non-diabetic group in the group treated with givinostat simultaneously with blood glucose control. In the thymus, TNF-α staining was observed throughout the cells in almost 100% of the cells in the diabetes mellitus untreated group and the group whose diabetes mellitus was not ameliorated. When TNF-α positive cells were present in the entire body, killer T cells that attack them were removed by negative selection as autoantigen-reactive T cells, such that it was considered that even when abnormal cells expressing TNF-α and proinsulin appeared in the body, these cells were not removed. When pancreatic islets were subjected to TNF-α immunostaining (DAB staining) (FIG. 22), almost no TNF-α positive cells were observed in the treatment success group of each of the streptozotocin induced diabetic mice with non-diabetic mice, insulin pellet + givinostat treatment (oral), and insulin pellet + givinostat treatment (intravenous). On the other hand, it was observed that a large amount of TNF-α positive cells migrated to the center of the pancreatic islets in the presence of blank pellet + givinostat treatment (oral) and in the presence of blank pellet + givinostat treatment (intravenous). This is considered to indicate that, without correcting hyperglycemia, the improvement of sugar uptake in pancreatic islets by the direct action of givinostat and the promotion of insulin cause a large amount of TNF-α positive cells derived from the bone marrow to gather by migration when β-cells start regeneration, such that the regeneration site is destroyed and inflammation is continued. In addition, only a few TNF-α positive cells were observed in the STZ only group (blank pellet + no givinostat treatment) as compared with the blank pellet + givinostat treatment. In this group, the insulin-producing cells were almost destroyed by STZ, and a niche for initiating regeneration was not formed. Therefore, it was considered that there was no migration of TNF-α positive cells derived from the bone marrow.

In diabetes mellitus, abnormal stem cells showing TNF-α positive/proinsulin positive are present in Vcam1 positive short-term hematopoietic stem cells in the bone marrow. Normal short-term hematopoietic stem cells include capillary progenitor cells of various organs, vascular niche cells, and also stem cells of organs. In the thymus, since there is a possibility that these cells are cells constituting the origin of the organ itself of the thymus, it is considered that these cells cannot migrate into the thymus under hyperglycemia in diabetes mellitus. Therefore, it is considered that killer T cells for attacking abnormal stem cells showing TNF-α positive/proinsulin positive in the bone marrow that have appeared in diabetes cannot be generated in the thymus. In diabetes mellitus, it is considered that the cells continue to survive as "diabetic stem cells" and are not cured only by blood glucose control.

### (Example 4)

An HDAC regulator and blood glucose control were combined to treat diabetes mellitus in NOD mice.

The treatment was started for NOD mice one week after confirmation of the onset of hyperglycemia. Insulin glargine was subcutaneously administered daily while the dosage was gradually reduced to 10 IU until day 3 after the start of treatment, 5 IU for 3 days thereafter, 2.5 IU for 6 days thereafter, and 0.75 IU for 12 days thereafter. 2 hours after administration of insulin glargine, 2.5 mg/kg of givinostat was orally administered daily to a mouse whose blood glucose level was measured and blood glucose lowering was confirmed. When the blood glucose level before administration of insulin glargine was lower than 200 mg/dl at longer than 8 weeks after the start of treatment, the administration of insulin glargine and givinostat on that day was discontinued. When the blood glucose level on the next day was also below 200 mg/dl, insulin glargine and givinostat were stopped, and then, the blood glucose level was measured weekly until week 4 to determine the diabetic state. When the blood glucose level did not fall below 200 mg/dl even after 8 weeks from the start of treatment, treatment was performed every day until 12 weeks, and the treatment was terminated at the time point after 12 weeks. When hypoglycemia appeared, blood glucose levels were measured 1 hour after intraperitoneal (300 µL) administration of 25% glucose solution, and this operation was repeated when hypoglycemia was still persistent. After it was confirmed that the blood glucose level was sufficiently increased (150 mg/dl or more), no further administration of the glucose solution was performed. When hypoglycemia was confirmed, the subsequent insulin was adjusted to 1/2 of the previous dose as necessary.

Diabetes mellitus in NOD mice was also cured.

### (Example 5)

Diabetes mellitus was treated by administering a combination of an HDAC regulator and a blood glucose control agent to db/db mice with developed diabetes mellitus.

Db/db mice were observed from week 6, and the time point at which the blood glucose level exceeded 300 mg/dl was defined as the onset of diabetes mellitus. In the db/db mice, insulin glargine was subcutaneously administered daily while the dosage was gradually reduced to 10 IU until day 3 after the start of treatment, 5 IU for 3 days thereafter, 2.5 IU for 6 days thereafter, and 0.75 IU for 12 days thereafter. 2 hours after administration of insulin glargine, 2.5 mg/kg of givinostat was orally administered daily to a mouse whose blood glucose level was measured and blood glucose lowering was confirmed. When the blood glucose level before administration of insulin glargine was lower than 200 mg/dl at longer than 8 weeks after the start of treatment, the administration of insulin glargine and givinostat on that day was discontinued. When the blood glucose level on the next day was also below 200 mg/dl, insulin glargine and givinostat were stopped, and then, the blood glucose level was measured weekly until week 4 to determine the diabetic state. When the blood glucose level did not fall below 200 mg/dl even after 8 weeks from the start of treatment, treatment was performed every day until 12 weeks, and the treatment was terminated at the time point after 12 weeks. When hypoglycemia appeared, blood glucose levels were measured 1 hour after intraperitoneal (300 µL) administration of 25% glucose solution, and this operation was repeated when hypoglycemia was still persistent. After it was confirmed that the blood glucose level was sufficiently increased (150 mg/dl or more), no further administration of the glucose solution was performed. When hypoglycemia was confirmed, the subsequent insulin was adjusted to 1/2 of the previous dose as necessary.

Diabetes mellitus in an obese mouse model was also cured.

### (Example 6)

Diabetes mellitus was treated by administering a combination of an HDAC regulator and a different type of blood glucose control agent to db/db mice with developed diabetes mellitus.

Db/db mice were observed from week 6, and the time point at which the blood glucose level exceeded 300 mg/dl was defined as the onset of diabetes mellitus. In db/db mice with leptin receptor deficiency, 1.0 mg/kg of dapagliflozin, an SGLT inhibitor, and 2.5 mg/kg of givinostat, an HDAC regulator, were orally administered daily for 8 weeks, after which the treatment was discontinued for 4 weeks and terminated.

Diabetes mellitus was also cured when a different type of blood glucose control agent was used.

### (Example 7)

Diabetes mellitus was similarly cured using, instead of givinostat, fingolimod (FTY720), trichostatin A, vorinostat, belinostat, panobinostat, resminostat, abexinostat, quisinostat, pracinostat, rocilinostat, nanatinostat, dacinostat, (N-hydroxy-7-2-naphthylthio)heptanomide, valproate, CG200745, scriptaid, KBH-A42, romidepsin, sodium butyrate, 4-phenylbutyric acid, SK-7041, WW437, entinostat, tacedinaline, mocetinostat, (pivaloyloxy)methyl butyrate, trapoxin, α-ketoamides, heterocyclic ketones, diallyl trisulfide, ricolinostat, Tubacin, azelaoyl-bis-hydroxamic acid, and m-carboxycinnamic acid bis-hydroxamide.

### (Example 8: Effect of thymectomy)

The effect of the thymus when STZ-DM mice were treated with a combination of an HDAC regulator and blood glucose control was examined. An overview of the experiment is illustrated in FIG. 23.

Streptozotocin (150 mg/kg) was tail vein-administered to C57BL/6 J mice (wild type, CLEA Japan, Inc., Osaka) to develop diabetes mellitus. After one month of free feeding, an insulin pellet was subcutaneously administered to these STZ-DM mice to normalize their blood glucose levels. The mice were divided into two groups, for one group of mice, the thymus was removed from the suprasternal fossa (thymectomy group), and for the other group of mice, an incision was made in the suprasternal fossa and closed immediately (control group). Givinostat (Cayman chemical, USA) was orally administered at 2.5 mg/kg to both the thymectomy group and the control group. Givinostat was administered once every three days.

Blood glucose levels of mice in both groups were measured weekly (FIG. 24). After the end of 8 weeks of treatment, perfusion fixation was performed on both groups of mice to confirm the presence or absence of thymus (FIG. 25).

### Results

As illustrated in FIG. 24, in the thymectomy group, the blood glucose level was significantly higher than that in the control group from week 4 or later when the effect of the insulin pellet started to decrease, and increased to about 400 mg/dL at the end of treatment. On the other hand, no increase in blood glucose levels was observed in the control group treated without thymectomy. As illustrated in FIG. 25, in the mice of the control group, the thymus atrophied by diabetes mellitus recovered to a normal level, but in the thymectomy group, the thymus did not recover even when the treatment was performed. It is considered from this that the function of the thymus is essential for the complete recovery of diabetes mellitus. In addition, in NOD mice as a model of type 1 diabetes mellitus developed by abnormal autoimmunity, similar effects were observed by thymectomy.

### (Note)

Although the present disclosure has been illustrated using preferred embodiments of the present disclosure as described above, it is understood that the scope of the present disclosure should be interpreted only by the claims. It is understood that the patents, patent applications, and other documents cited in the present specification are to be incorporated by reference in the present specification in the same manner as the contents is specifically described in the present specification.

The present application claims priority to Japanese Patent Application No. 2021-177845 filed to the Japan Patent Office on October 29, 2021. The entire content thereof is incorporated herein by reference.

### Industrial Applicability

The present disclosure provides a diabetes mellitus treatment by combining control of blood glucose and HDAC regulation, which provides a new approach for diabetes mellitus treatment.

## Claims

1. A composition comprising a histone deacetylase (HDAC) regulator for a treatment and/or prevention of diabetes mellitus and/or a disease, disorder, and/or symptom associated with diabetes mellitus in a subject, wherein the composition is administered in a controlled blood glucose state of the subject.

2. A composition comprising a histone deacetylase (HDAC) regulator for a treatment and/or prevention of diabetes mellitus and/or a disease, disorder, and/or symptom associated with diabetes mellitus in a subject, wherein the composition is administered in combination with a blood glucose control treatment.

3. The composition according to claim 1 or 2, wherein the HDAC regulator inhibits at least one of class I, class II, and class IV HDACs.

4. The composition according to any one of claims 1 to 3, wherein the HDAC regulator comprises at least one selected from the group consisting of fingolimod, givinostat, vorinostat (suberoylanilide hydroxamic acid, SAHA), belinostat, panobinostat, resminostat, abexinostat, mocetinostat, trichostatin A (TSA), romidepsin, apicidin, chidamide, entinostat, tacedinaline, tucidinostat, pracinostat (SB939), quisinostat, tefinostat, rocilinostat, fimepinostat, nanatinostat, domatinostat, trapoxin (TPX), cyclic hydroxamic acid-containing peptide 1 (CHAP1), valproate (VPA), sodium butyrate (NaBu), 4-phenylbutyric acid, OBP-801, ME-344, CG200745, MPT0E014, RGFP966 (MCE, HY-13909), SK-7041, scriptaid, MC1568, MHY2256, ricolinostat (ACY-1215), Tubacin (S2239), Tubathian A, Tubastatin A, Pojamide, AN7, CUDC-101, KD5170, R36465, SOU-HDAC42, LBH589, SFN, phenylhexyl isothiocyanate, dacinostat (LAQ824), m-carboxycinnamic acid bis-hydroxamide (CBHA), azelaoyl-bis-hydroxamic acid (ABHA) (N,N'-dihydroxynonanediamide), AR-42, suberoyl bis-hydroxamic acid (SBHA), (pivaloyloxy)methyl butyrate (pivanex, AN-9), diallyl trisulfide (DATS), PCI-24781, nicotinamide, tenovin-1, tenovin-6, sirtinol, EX-527, HBI-8000, kevetrin, CHR-3996, 4SC202, WW437, α-ketoamides, heterocyclic ketones, cambinol, I-7ab, C149, depudecin, SEN 196, COMPOUND6J, JGB 1741, JQ1, I-BET151, BY27, CUDC907, DWP0016, MC1568, W2, RGFP109, tinostamustine (EDO-S101), J22352, M344, ITF-3056, TMP195 (TFMO 2), MI192, Santacruzamate A(CAY10683), ACY-738, BML-281 (CAY10603), LTB2, CKD-506, WK2-16, PCI-34051, ESM-HDAC528, Merck60, (N-hydroxy-7-2-naphthylthio)heptanomide (HNHA), KBH-A42, AGK2, sulforaphane, MS-275, resveratrol, APHA, curcumin, remetinostat, citarinostat, dacinostat, droxinostat, BRD3308, nimbolide, betaine, and chrysophanol.

5. The composition according to any one of claims 1 to 3, wherein the HDAC regulator comprises at least one selected from the group consisting of fingolimod, givinostat, trichostatin A, vorinostat, belinostat, panobinostat, resminostat, abexinostat, quisinostat, pracinostat, rocilinostat, nanatinostat, dacinostat, (N-hydroxy-7-2-naphthylthio)heptanomide, valproate, CG200745, scriptaid, KBH-A42, romidepsin, sodium butyrate, 4-phenylbutyric acid, SK-7041, WW437, entinostat, tacedinaline, mocetinostat, (pivaloyloxy)methyl butyrate, trapoxin, α-ketoamides, heterocyclic ketones, diallyl trisulfide, ricolinostat, Tubacin, azelaoyl-bis-hydroxamic acid, and m-carboxycinnamic acid bis-hydroxamide.

6. The composition according to any one of claims 1 to 5, wherein the control of blood glucose is performed by at least one selected from the group consisting of administration of a hypoglycemic agent, dietary restriction, and nutrient infusion control.

7. The composition according to claim 6, wherein the hypoglycemic agent is selected from the group consisting of insulin, an insulin analog, a biguanide, an insulin secretagogue, a fast-acting insulin secretagogue, an incretin-related drug, a sugar absorption regulator, an insulin resistance-ameliorating agent, a urine glucose evacuant, and a combination of two or more thereof.

8. The composition according to any one of claims 1 to 7, wherein the control of blood glucose comprises controlling fasting blood glucose to less than 126 mg/dL, controlling a blood glucose level at 2 hours of an oral glucose tolerance test to less than 200 mg/dL, or controlling a casual blood glucose level to less than 200 mg/dL.

9. The composition according to any one of claims 1 to 8, wherein the composition is used for treating diabetes mellitus and/or a disease, disorder, and/or symptom associated with diabetes mellitus.

10. The composition according to any one of claims 1 to 9, wherein the disease, disorder, and/or symptom comprises diabetic complications.

11. The composition according to any one of claims 1 to 10, wherein the disease, disorder, and/or symptom is selected from the group consisting of a neurological disorder, nephropathy, a hepatic disorder, retinopathy, fatty liver, a gastrointestinal disorder, delayed fracture healing, an eating disorder, and a skin disorder.

12. The composition according to any one of claims 1 to 11, wherein the subject is diagnosed as having abnormal hematopoietic stem cells (HSCs) or cells derived therefrom.

13. The composition according to claim 12, wherein the abnormal hematopoietic stem cells have at least one of variable levels of HDAC and increased levels of CD106.
